# EUROPEAN PATENT APPLICATION

(11) **EP 4 306 547 A1**
(43) Date of publication of application: **17.01.2024**
(21) Application number: 22184566.2
(22) Date of filing: 13.07.2022
(51) Int. Cl.: C08B 37/02, C08L 5/02, C08J 3/075, A61K 47/36, A61L 27/52

(54) **HYDROGELS FOR DRUG DELIVERY**

(71) Applicant: Adocia, 69003 Lyon (FR)
(72) Inventor: GEISSLER, Alexandre, 69330 Meyzieu (FR); LAURENT, Nicolas, 01700 Miribel (FR); PLANCQ, Baptiste, 01700 Saint Maurice de Beynost (FR); SOULA, Gérard, 69330 Meyzieu (FR); BESNARD, Romain, 69270 Couzon au Mont d'or (FR); DAUTY, Emmanuel, 69004 Lyon (FR)
(74) Representative: Tripoz, Inès

(57) **Abstract**

The invention concerns cross-linked dextran polymer Dx bearing anionic groups wherein the at least divalent radical L(-)i is covalently bound to the dextran polymer backbone with i-W-radicals, wherein,
- L(-)ᵢ is a linear or branched polyether
- i is the valence of L and the number of-W-radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

It also concerns the dextran polymers before the crosslinking reaction and the process to synthetize a cross-linked dextran polymer according to the invention, into the form of a hydrogel.

It also concerns a hydrogel comprising the crosslinked dextran polymer of the invention and/or a hydrogel that further comprises an API.

It also concerns therapeutic use of the hydrogel according to the invention as an implant for drug delivery.

## Description

The domain of the invention is therapy, in particular drug delivery. More particularly the invention is about an implant comprising a hydrogel which may incorporate active principles, such as peptides, hormones, proteins or small molecules.

The aim is to prevent, treat or cure disease by administration of a suitable dose of an active pharmaceutical ingredient (API). The invention is also about a crosslinked polymer, its precursors, a process for obtaining the crosslinked polymer and a process for obtaining a hydrogel, in particular a hydrogel containing API.

Hydrogels can be used as controlled drug or active pharmaceutical ingredient release systems.

Hydrogels comprise or consist of polymers that are crosslinked in a 3D network. They can either be natural or synthetic, homopolymers or copolymers. They have the ability to absorb and retain large amounts of water. This is known as the swelling of hydrogels.

In order to have a system which may be an implant able to deliver active principle on the long run, many features have to be obtained.

Among these features may be cited:
a low degradability, in particular a low biodegradability, or no biodegradability,
or a good in vivo stability, in order for the implant API to beretrievable,
   - a good mitigation of the foreign body response, or a good biocompatibility, in particular a low cytotoxicity and a good local tolerance.

In order to be used as controlled release systems for API, hydrogels must have particular characteristics so as to exhibit all or part of the desired properties such as disclosed above as well as good mechanical and rheological properties.

Among the rheological and mechanical properties that are of high interest for the hydrogel may be cited:
- a good homogeneity, which can be linked to a good transparency or translucency,
- appropriate resistance and flexibility toward stress and strain mechanics, in particular when being handled and implanted,
- a defined mesh size to control the release rate of the API
- a good stability in vivo, i.e. resistance to hydrolytic, enzymatic or oxidative degradation.

Among parameters which can give indications on the rheological and mechanical desired properties may be cited:
- tan δ (called loss tangent) which gives indication on mechanical properties,
- G', which gives indication on the elastic modulus (stiffness), and on the mesh size,
- compression and/or traction deformation at break, which gives indication on the elasticity and resistance of the hydrogel,
- swellability, which gives indications on the water content, dimensions and mechanical properties.

Among the problems to be solved is obtaining a hydrogel with properties allowing:
- a manipulation for implanting the hydrogel without breaking it, and/or
- a gel to remain in place after implantation, for example allowing the hydrogel not to get folded after implantation and/or to be immobilized relative to the tissue on which it is implanted, or
- An implantation of the gel via a minimally invasive procedure, in particular a subcutaneous injection.

Another problem to be dealt with relates to the sedimentation of API during the crosslinking leading to gelation.

The suitability of the hydrogel depends on its bulk structure, thus important parameters used to characterize the network structure of the hydrogel according to the invention are the polymer volume fraction in the swollen state, the molecular weight of the polymer chain between two neighboring cross-linking points, and the corresponding mesh size.

The problem is solved by the provision of a new cross-linked dextran polymer, bearing anionic groups, wherein at least two saccharidic units of dextran belonging to two different polymer chains are covalently crosslinked by at least one central linker radical L(-)ᵢ, this at least radical being a at least divalent linear, branched or cyclic alkyl radical comprising at least a polyethylene glycol chain.

The properties of this family of hydrogels are tunable and tailorable to the applications by choosing and adapting the cross-linking reaction conditions, the substitution degree and molecular weight of the dextrans and cross-linkers.

The cross-linked dextran polymer according to the invention is a dextran polymer Dx bearing anionic groups wherein the at least divalent radical L(-)ᵢ is covalently bound to the dextran polymer backbone with i-W-radicals, wherein,
- L(-)ᵢ is a linear or branched polyether
- i is the valence of L and the number of-W-radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

In an embodiment the implant is obtained via molding.

In another embodiment the implant is obtained by injection of precursors of the hydrogel with an API.

When referring to an API, whether specified as a particular compound or a compound class the term used is intended to encompass not only the specified molecular entity or entities but also pharmaceutically acceptable, pharmacologically active analogs and derivatives thereof, including, but not limited to, salts, esters, prodrugs, conjugates, active metabolites, crystalline forms, enantiomers, stereoisomers, and other such derivatives, analogs, and related compounds.

In an embodiment the API is chosen among the progestogens. Non limiting examples of progestogens include desogestrel, dienogest, drospirenone, ethisterone, etonogestrel, gestodene, levonorgestrel, medroxyprogesterone, megestrol, norethindrone, norgestimate, and esters of any of the foregoing, when the compound allows for esterification (e.g., medroxyprogesterone acetate, megestrol acetate, norethindrone acetate, etc.).

In an embodiment the API is chosen among the cancer drugs. Non-limiting examples of cancer drugs include coxorubicin, paclitaxel, camptothecin, docetaxel, pemetrexed, curcumin, gemcitabine, dabrafenib, dexamethasone, gefitinib, lenvatinib, methotrexate, thalidomide, vinblastine, vincristine, cyclophosphamide, ifosfamide, glyciphosphoramide, nimustine, carmustine, comustine, 5-fluorouracil, doxifluridine, mercaptopurine, cisplatin, and combinations thereof.

In an embodiment the API is chosen among corticosteroids. Non-limiting examples of the corticosteroids include cortisone, prednisone, prednisolone, methylprednisolone, dexamethasone, betamethasone, hydrocortisone, and combinations thereof.

In an embodiment the API is chosen among hormones. Non- limiting examples hormones include alclometasone, prednisone, dexamethasone, triamcinolone, cortisone, fludrocortisone, dihydrotachysterol, oxandrolone, oxabolone, testosterone, nandrolone, diethylstilbestrol, ethinyl estradiol, norethisterone, medroxyprogesterone acetate, hydroxyprogesterone caproate, estrogen, estradiol, estriol, estrone, cortisol, 11 -deoxy cortisol, aldosterone, corticosterone, 11-deoxycorti-costerone, aldosterone, progestin, pregnenolone, progesterone, 17a-hydroxy progesterone, 17a-hydroxy pregnenolone, dehydroepiandrosterone, androstenedoil, androstenedione, dihydrotestosterone, melatonin, thyroxine, and combinations thereof.

In an embodiment the API is chosen among drugs for pain relief. Non-limiting examples of drugs for pain relief include NSAIDS, opioids or local anesthetics.

Non-limiting examples of NSAIDS include Indomethacin, Sulindac, Etodolac, Tolmetin, Ketorolac, Oxaprozin, Fenoprofen, Flurbiprofen, Ibuprofen, Ketoprofen, Naproxen, Nambumetone, Meclofenamate, Diclofenac, Piroxicam, Meloxicam, Celecoxib, Rofecoxib, Valdecoxib, Aspirin, and combinations thereof.

Non-limiting examples of opioids include Fentanyl, Alfentanil, Sufentanil, Remifentanil, Methadone, and combinations thereof.

Non-limiting examples of local anesthetics include Dibucaine, Bupivacaine, Lidocaine, Procaine, Mepivacaine, Rapivacaine, and combinations thereof.

In an embodiment the API is chosen among anti-VIH drugs. Non-limiting examples of anti-VIH drugs include islatravir, cabotegravir, rilpivirine, dapivirine, tenofovir alafenamide, lenacapavir, elsulfavirine and combinations thereof.

In an embodiment the API is chosen among anti-malaria drugs. Non-limiting examples of anti-malaria drugs are Atovaquone, proguanil, ivermectin and combinations thereof.

In an embodiment the API is chosen among anti-tuberculosis drugs and anti HPV (Human Papillomavirus).

In an embodiment, the API is incorporated in particulate form.

In an embodiment, the API is hydrophobic or hydrophobized in order to be in a particulate form in suspension into the hydrogel of the invention.

The hydrophobization of hydrophilic API could be done by encapsulation methods, controlled precipitation with a hydrophobic excipient, emulsification and other classical methods used in galenic.

In an embodiment-W-comprises at most 60 carbon atoms.

In an embodiment-W-comprises at most 60 carbon atoms without counting any -CH₂-CH₂O- radicals.

In an embodiment-W-comprises at most 50 carbon atoms.

In an embodiment-W-comprises at most 50 carbon atoms without counting any -CH₂-CH₂O- radicals.

In an embodiment-W-comprises at most 40 carbon atoms.

In an embodiment-W-comprises at most 40 carbon atoms without counting any -CH₂-CH₂O- radicals.

In an embodiment-W-comprises at most 30 carbon atoms.

In an embodiment-W-comprises at most 30 carbon atoms without counting any -CH₂-CH₂O- radicals.

In an embodiment-W-comprises at most 20 carbon atoms.

In an embodiment-W-comprises at most 20 carbon atoms without counting any -CH₂-CH₂O- radicals.

In an embodiment-W-comprises at most 10 carbon atoms.

In an embodiment-W-comprises at most 10 carbon atoms without counting any -CH₂-CH₂O- radicals.

In an embodiment-W-comprises at most 10 oxygen atoms.

In an embodiment-W-comprises at most 10 oxygen atoms without counting any -CH₂-CH₂O- radicals.

In an embodiment-W-comprises at most 5 oxygen atoms.

In an embodiment-W-comprises at most 5 oxygen atoms without counting any -CH₂-CH₂O- radicals.

The crosslinked dextran hydrogel according to the invention is a dextran polymer wherein the central-linker L(-)ᵢ is a linear, or a branched polyethylene glycol (PEG) radical.

By branched PEG is meant various PEG arms connected by a linear, branched, or cyclic alkyl, or by an aromatic, comprising between 2 to 20 carbon atoms and may comprise heteroatoms such as nitrogen, oxygen, or sulphur.

In one embodiment, the crosslinked dextran hydrogel according to the invention is a dextran polymer wherein the central-linker L(-)ᵢ is a branched PEG radical which possesses at most 8 arms.

In an embodiment, the central-linker L(-)ᵢ is a PEG chosen among the PEG of formula I :

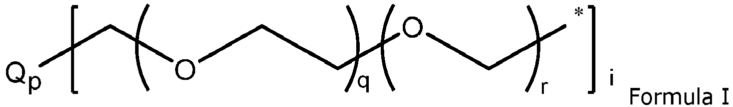

Wherein:
- i is an integer comprised from 2 to 8 (2 ≤ i ≤ 8)
- p is an integer equal to 0 or 1, and if i=2 then p=0
- q is an integer comprised from 8 to 1000 (8 ≤ q ≤ 1000)
- r is an integer equal to 0 or 1
- Q is either a carbon atom, or a linear, branched, or cyclic alkyl chain, or an aromatic, comprising 2 to 10 carbon atoms and may comprise heteroatoms such as nitrogen, oxygen, or sulphur
- the ^{∗} represents the sites of f₄, which is an amine function, or an ether, or a thioether function, or an amide function, or a carbamate function or a carbon-nitrogen covalent bond, or a carbon-aromatic carbon covalent bond, or a carbon-carbon covalent bond if the crosslinking process is made by a Native Chemical Ligation (NCL).

In an embodiment, the cross-linked dextran polymer according to the invention is a dextran polymer wherein L(-)ᵢ is a radical issued from a linear or branched mercaptopolyethyleneglycol comprising at least 2 sulfur atoms and comprising at most 8 arms, which
- number-average molecular weight (Mn) is comprised from 500 to 40 000 g/mol (500 ≤ Mn ≤ 40 000 g/mol) or
- polymerisation degree (DP) is comprised from 8 to 1000 (8 ≤ DP ≤ 1000).

In an embodiment, the cross-linked dextran polymer according to the invention is a dextran polymer wherein L(-)ᵢ is a radical issued from a linear or branched mercaptopolyethyleneglycol comprising at least 2 sulfur atoms and comprising at most 8 arms, which
- Mn is comprised from 1000 to 25 000 g/mol (1000 ≤ Mn ≤ 25 000 g/mol) or
- polymerisation degree (DP) is comprised from 15 to 600 (15 ≤ DP ≤ 600).

In an embodiment, the cross-linked dextran polymer according to the invention is a dextran polymer wherein L(-)ᵢ is a radical according to formula I, issued from the thiol polyethylene glycols or mercaptopoly(oxyethylenes) cited in the following table:

| Chemical Name | i | Mn (kg/mol) |
|---|---|---|
| Poly(ethylene glycol) dithiol | 2 | 10 |
| Poly(ethylene glycol) dithiol | 2 | 3.4 |
| Poly(ethylene glycol) dithiol | 2 | 1 |
| Pentaerythritol tetra(mercaptoethyl) polyoxyethylene | 4 | 5.2 |
| Pentaerythritol tetra(mercaptoethyl) polyoxyethylene | 4 | 20 |
| tripentaerythritol octa(mercaptoethyl) polyoxyethylene | 8 | 20 |

In an embodiment, the cross-linked dextran polymer according to the invention is a dextran polymer wherein L(-)ᵢ is a radical according to formula I issued from a pentaerythritol tetra(mercaptoethyl) polyoxyethylene, CAS# 188492-68-4.

In an embodiment, the cross-linked dextran polymer according to the invention is a dextran polymer wherein L(-)ᵢ is a radical according to formula I issued from a linear (mercaptoethyl)polyoxyethylene, CAS# 68865-60-1.

In an embodiment, the cross-linked dextran polymer according to the invention is a dextran polymer wherein L(-)ᵢ is a radical according to formula I, issued from a pentaerythritol poly(oxyethylene) azide cited in the following table:

| Chemical Name | i | Mn (kg/mol) |
|---|---|---|
| 2-arm PEG azide (Polyoxyethylene bis(azide)) | 2 | 5 |
| 2-arm PEG azide | 2 | 10 |
| 4-arm PEG azide, pentaerythritol core | 4 | 20 |
| 4-arm PEG azide, pentaerythritol core | 4 | 40 |

In an embodiment, the cross-linked dextran polymer according to the invention is a dextran polymer wherein L(-)ᵢ is a radical according to formula I, issued from a pentaerythritol poly(dibenzocyclooctyne) polyoxyethylene cited in the following table:

| Chemical Name | i | Mn (kg/mol) |
|---|---|---|
| 2-arm PEG DBCO Dibenzocycloctyne-PEG-Dibenzocycclotyne | 2 | 5 |
| 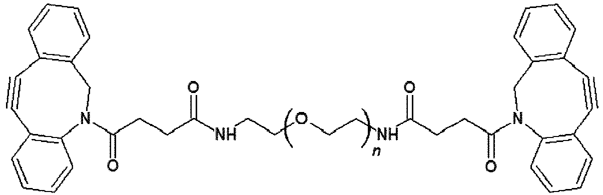 | | |
| 2-arm PEG DBCO | 2 | 10 |
| 4-arm PEG DBCO, pentaerythritol core | 4 | 20 |
| 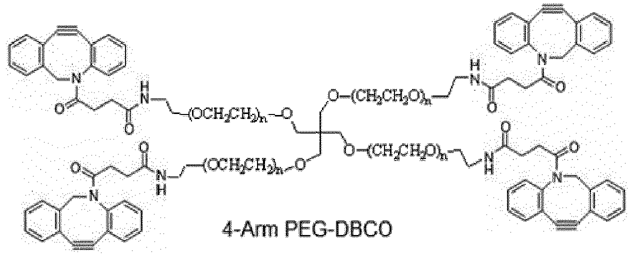 | | |
| 4-arm PEG DBCO, pentaerythritol core | 4 | 40 |

The hydroxyl functions of the dextran polymer Dx- can be functionalised by at least one specific anionic group such as: alkyl carboxylate, sulphate anions, or sulfonate anions, or phosphate anions, or phosphonate anions.

In one embodiment, the hydroxyl functions of the dextran polymer Dx can be functionalised by sulphate anions in salified form, and optionally by alkyl carboxylate derivatives in salified form.

In another embodiment, the hydroxyl functions of the dextran polymer Dx can be functionalised by sulfonate anions in salified form, and optionally by alkyl carboxylate derivatives in salified form.

In another embodiment, the hydroxyl functions of the dextran polymer backbone Dx-, can be functionalized by phosphate anions in salified form, and optionally by alkyl carboxylate derivatives in salified form.

In another embodiment, the hydroxyl functions of the dextran polymer Dx can be functionalised by phosphonate anions in salified form, and optionally by alkyl carboxylate derivatives in salified form.

In another embodiment, the hydroxyl functions of the dextran polymer Dx can be functionalised by alkyl carboxylate derivatives in salified form.

The specific anionic groups defined previously are chosen among the groups of formula II:

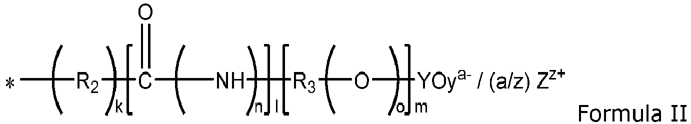

Wherein:
- ^{∗} represents the link to the O atoms of the dextran to form an ether function.
- y=2 or 3.
- When y=2, alkyl carboxylate derivatives, then:
   ∘ Y=C and a=1.
   ∘ k=1, l=0 and m=0.
   ∘ R2=Alkyl.
- When y=3, anionic group, then:
   ∘ Y=S and a=1, or Y=P and a=2.
   ∘ k=0 or 1.
   ∘ l=0 or 1.
   ∘ m=0 or 1.
   ∘ n=1 or 2, in particular n = 1,
   ∘ o=0 or 1.
   ∘ if l=1 then m=1.
   ∘ R₃=linear, branched, or cyclic alkyl which may contain one heteroatom such as nitrogen, or aromatic, or PEG.
   ∘ R2=Alkyl.
- And, Z is a counter ion, which can be an alkali metal and z=1, or which can be an alkaline earth metal and z=2.

In a preferred embodiment, the dextran backbone, Dx-, can be functionalised by sulphate anions in salified form, and optionally by alkyl carboxylate derivatives in salified form.

In another preferred embodiment, the dextran backbone, Dx-, can be functionalised by alkyl sulfonate anions in salified form, and optionally by alkyl carboxylate derivatives in salified form.

In another preferred embodiment, the dextran backbone, Dx-, can be functionalised by sulfonate anions in salified form, supported by an alkyl chain comprising a dimethyl-ammonium cation, and optionally by alkyl carboxylate derivatives in salified form.

In another preferred embodiment, the dextran backbone, Dx-, can be functionalised by alkyl carboxylate derivatives in salified form.

In an embodiment, the cross-linked dextran polymer bearing anionic groups according to the invention is a dextran polymer wherein the dextran polymer backbone is according to formula III, 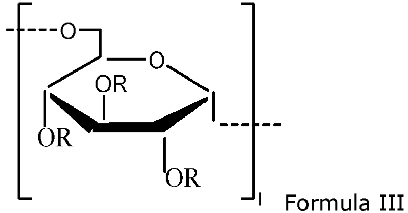 wherein R is chosen among
- -H, a anionic group of formula II, or a -W- radical bearing a L(-)ᵢ crosslinker,
- i is comprised from 20 to 5000 (20 ≤ i ≤ 5000),
- -W- and L(-)ᵢ radicals having the previously defined meanings.

In an embodiment, the cross-linked dextran polymer according to the invention is a dextran polymer wherein the dextran polymer backbone is a dextran having a weight average molecular weight (Mw) comprised from 5 to 1000 kDa before crosslinking and substitution.

In other words, the cross-linked dextran polymer according to the invention is obtained after substitution and crosslinking of a native dextran polymer having a weight average molecular weight (Mw) comprised from 5 to 1000 kDa.

In an embodiment, the cross-linked dextran polymer according to the invention is a dextran polymer wherein the dextran polymer backbone is a dextran having a weight average molecular weight (Mw) comprised from 5 to 250 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 100 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 50 kDa before cross-linking and substitution.

In an embodiment, the cross-linked dextran polymer according to the invention is a dextran polymer wherein the dextran polymer backbone is a dextran having a weight average molecular weight (Mw) comprised from 5 to 25 kDa before cross-linking and substitution.

In an embodiment, the cross-linked dextran polymer according to the invention is a dextran polymer wherein the dextran polymer backbone is a dextran having a weight average molecular weight (Mw) comprised from 250 to 1000 kDa before crosslinking and substitution.

In an embodiment, the cross-linked dextran polymer according to the invention is a dextran polymer wherein the dextran polymer backbone is a dextran having a weight average molecular weight (Mw) comprised from 10 to 500 kDa before crosslinking and substitution.

In an embodiment, the cross-linked dextran polymer according to the invention is a dextran polymer wherein the dextran polymer backbone is a dextran having a weight average molecular weight (Mw) comprised from 20 to 500 kDa before crosslinking and substitution.

In an embodiment, the cross-linked dextran polymer according to the invention is a dextran polymer wherein the dextran polymer backbone is a dextran having a weight average molecular weight (Mw) comprised from 20 to 100 kDa before crosslinking and substitution.

In an embodiment, the cross-linked dextran polymer according to the invention is a dextran polymer wherein the dextran polymer backbone is a dextran having a weight average molecular weight (Mw) comprised from 20 to 50 kDa before cross-linking and substitution.

In an embodiment, the cross-linked dextran polymer according to the invention is a dextran polymer wherein the dextran polymer backbone is a dextran having a weight average molecular weight (Mw) comprised from 40 to 250 kDa before crosslinking and substitution.

In an embodiment, the cross-linked dextran polymer according to the invention is a dextran polymer wherein the dextran polymer backbone is a dextran having a weight average molecular weight (Mw) comprised from 40 to 100 kDa before crosslinking and substitution.

In an embodiment, the cross-linked dextran polymer according to the invention is a cross-linked dextran polymer wherein the degree of substitution (DS₁) of the dextran backbone with the a -W- radical bearing a L(-)ᵢ crosslinker is comprised in the range from 0.001 to 0.4 (0.001 ≤ DS₁ ≤ 0.4).

In an embodiment, the cross-linked dextran polymer according to the invention is a cross-linked dextran polymer wherein the degree of substitution (DS₁) of the dextran backbone with the -W- radical bearing a L(-)ᵢ crosslinker is comprised in the range from 0.01 to 0.4 (0.01 ≤ DS₁ ≤ 0.4).

In an embodiment, the cross-linked dextran polymer according to the invention is a cross-linked dextran polymer wherein the degree of substitution (DS₁) of the dextran backbone with the -W- radical bearing a L(-)ᵢ crosslinker is comprised in the range from 0.05 to 0.4 (0.05 ≤ DS₁ ≤ 0.4).

In an embodiment, the cross-linked dextran polymer according to the invention is a cross-linked dextran polymer wherein the degree of substitution (DS₁) of the dextran backbone with the -W- radical bearing a L(-)ᵢ crosslinker is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 5 to 250 kDa before crosslinking and substitution and the degree of substitution (DS₁) of the dextran backbone with the -W- radical bearing a L(-)ᵢ crosslinker groups is comprised in the range from 0.1 to 0.4 (0.1 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) is from 20 to 100 kDa before crosslinking and substitution and the degree of substitution (DS₁) of the dextran backbone with the -W- radical bearing a L(-)ᵢ crosslinker groups is comprised in the range from 0.2 to 0.4 (0.2 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 20 to 100 kDa before cross-linking and substitution and the degree of substitution (DS₁) of the dextran backbone with the -W- radical bearing a L(-)ᵢ crosslinker is comprised in the range from 0.2 to 0.3 (0.2 ≤ DS₁ ≤ 0.3).

In an embodiment, the cross-linked dextran polymer according to the invention is a cross-linked dextran polymer wherein the dextran polymer backbone is a dextran having a weight average molecular weight (Mw) is from 250 to 1000 kDa before crosslinking and substitution and the degree of substitution (DS₁) of the dextran backbone with the -W- radical bearing a L(-)ᵢ crosslinker is comprised in the range from 0.001 to 0.4 (0.001 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with the -W- radical bearing a L(-)ᵢ crosslinker is comprised in the range from 0.01 to 0.4 (0.01 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with the -W- radical bearing a L(-)ᵢ crosslinker is comprised in the range from 0.05 to 0.4 (0.05 ≤ DS₁ ≤ 0.4).

In an embodiment, the cross-linked dextran polymer according to the invention is a cross-linked dextran polymer wherein the dextran polymer backbone is a dextran having a weight average molecular weight (Mw) from 250 to 1000 kDa before crosslinking and substitution and the degree of substitution (DS₁) of the dextran backbone with the -W- radical bearing a L(-)ᵢ crosslinker groups is comprised in the range from 0.1 to 0.4 (0.1 ≤ DS₁ ≤ 0.4).

In an embodiment, the cross-linked dextran polymer according to the invention is a cross-linked dextran polymer wherein the degree of substitution (DS₄) of the dextran backbone with a radical of formula II is comprised in the range from 0.5 to 3 (0.5 ≤ DS₄ ≤ 3).

In an embodiment, the cross-linked dextran polymer according to the invention is a cross-linked dextran polymer wherein the degree of substitution (DS₄) of the dextran backbone with a radical of formula II is comprised in the range from 1 to 2.75 (1 ≤ DS₄ ≤ 2.75).

In an embodiment, the cross-linked dextran polymer according to the invention is a cross-linked dextran polymer wherein the degree of substitution (DS₄) of the dextran backbone with a radical of formula II is comprised in the range from 1.5 to 2.5 (1.5 ≤ DS₄ ≤ 2.5).

In an embodiment, the cross-linked dextran polymer according to the invention is a cross-linked dextran polymer wherein the degree of substitution (DS₄) of the dextran backbone with a radical of formula II is comprised in the range from 1.75 to 2.25 (1.75 ≤ DS₄ ≤ 2.25).

In an embodiment, the cross-linked dextran polymer according to the invention is a cross-linked dextran polymer wherein the degree of substitution of carboxylate (DS_{c}) of the dextran backbone is comprised in the range from 0.2 to 3 (0.2 ≤ DS_{c} ≤ 3).

In an embodiment, the cross-linked dextran polymer according to the invention is a cross-linked dextran polymer wherein the degree of substitution of carboxylate (DS_{c}) of the dextran backbone is comprised in the range from 0.3 to 2.5 (0.3 ≤ DS_{c} ≤ 2.5).

In an embodiment, the cross-linked dextran polymer according to the invention is a cross-linked dextran polymer wherein the degree of substitution of sulfate, sulfonate, phosphate, phosphonate (DS₃) of the dextran backbone is comprised in the range from 0.2 to 2.5 (0.2 ≤ DS₃ ≤ 2.5).

In an embodiment, the cross-linked dextran polymer according to the invention is a cross-linked dextran polymer wherein the degree of substitution of sulfate, sulfonate, phosphate, phosphonate (DS₃) of the dextran backbone is comprised in the range from 0.3 to 2.0 (0.3 ≤ DS₃ ≤ 2.0).

In an embodiment, the cross-linked dextran polymer according to the invention is a cross-linked dextran polymer having a molar ratio (DC) between the molar concentration of the -W- radical and the molar concentration of the reactive functions of the cross-linker L(-)ᵢ comprised in a range from 0.5 to 1.5 (0.5 ≤ DC ≤ 1.5).

In an embodiment, the cross-linked dextran polymer according to the invention is a cross-linked dextran polymer having a molar ratio between the molar concentration of the -W- radical and the molar concentration of the reactive functions of the cross-linker L(-)ᵢ is comprised in a range from 0.8 to 1.2 (0.8 ≤ DC ≤ 1.2).

In an embodiment, the cross-linked dextran polymer according to the invention is a cross-linked dextran polymer having a molar ratio between the molar concentration of the -W- radical and the molar concentration of the reactive functions of the cross-linker L(-)ᵢ is comprised in a range from 0.9 to 1.1 (0.9 ≤ DC ≤ 1.1).

In an embodiment, the cross-linked dextran polymer according to the invention is a cross-linked dextran polymer having a molar ratio between the molar concentration of the -W- radical and the molar concentration of the reactive functions of the cross-linker L(-)ᵢ is comprised in a range from 0.95 to 1.05 (0.95 ≤ DC ≤ 1.05).

In an embodiment, the cross-linked dextran polymer according to the invention is a cross-linked dextran polymer having a molar ratio between the molar concentration of the -W- radical and the molar concentration of the reactive functions of the cross-linker L(-)ᵢ is 1 (DC = 1).

In an embodiment, the cross-linked dextran polymer according to the invention is a cross-linked dextran polymer obtained from a reaction between the reactive function of the-W-precursor and the reactive function of the (L)i precursor where reactive function are present in the same concentration (DC = 1) and are comprised in a range going from 5 to 25 mM.

In an embodiment they are comprised in the range of 5 to 10 mM.

In an embodiment they are comprised in the range of 10 to 15 mM.

In an embodiment they are comprised in the range of 15 to 20 mM.

In an embodiment they are comprised in the range of 20 to 25 mM.

In an embodiment -W- is chosen among the radicals of formula IV. 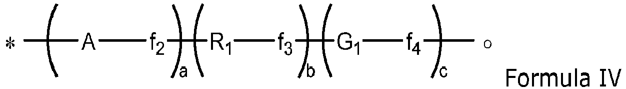 Wherein
- ^{∗} represents the site of f₁ and ° represents the site of attachment with L.
- a is an integer equal to 0 or 1.
- b is an integer equal to 0 or 1.
- c is an integer equal to 0 or 1.
- In one embodiment a=0, f₁ is an ether function, or a carbamate function.
- In one embodiment a=1,
   ∘ the divalent radical -A- is a linear, -(CH₂)ₙ₁- with n₁ an integer comprised from 1 to 7 (1 ≤ n₁ ≤ 7), branched, or cyclic alkyl derivative. It may also be branched by at least one hydroxyl group, -CH₂-CH(OH)-(CH₂)ₙ₂- with n₂ an integer comprised from 1 to 5 (1 ≤ n₂ ≤ 5); f₁ is an ether function, or a carbamate function, and f₂ is an amide function.
      Or,
   ∘ the divalent radical -A- is a linear polyether (PEG) derivative; f₁ is an ether function, or a carbamate function, and f₂ is an amide function. Or,
   ∘ in another embodiment the divalent radical -A- is a 4-Alkyl-1,4-triazole derivative or a 4-PEG-1,4-triazole derivative; f₁ is an ether function, or a carbamate function, and f₂ is a carbon-nitrogen covalent bond.
      Or,
   ∘ in another embodiment the divalent radical -A- is a 1-Alkyl-1,4-triazole derivative or a 1-PEG-1,4-triazole derivative; f₁ is an ether function, or a carbamate function, and f₂ is a carbon-aromatic carbon covalent bond.
- The divalent radical -R₁- is a linear, branched, or cyclic alkyl derivative, and/or an aromatic derivative, and/or a polyether (PEG) derivative, which can contain heteroatoms such as nitrogen, oxygen, or sulphur.
   ∘ If b=0, then f₁ is an ether function, or a carbamate function.
   ∘ If b=1, then f₁ is an ether function, or a carbamate function, and f₃ is an amide function, or an amine function, or an ether function, or a thioether function, or a carbamate function, or a carbon-nitrogen covalent bond, or a carbon-aromatic carbon covalent bond or a carbon-carbon covalent bond if the crosslinking process is made by a Native Chemical Ligation (NCL).
- The divalent radical -G₁- is a linear, branched, or cyclic alkyl derivative, or an aromatic derivative, which can contain heteroatoms such as: at most 5 nitrogen atoms, at most 10 oxygen atoms, at most 5 sulphur atoms, or at most one phosphorus atom. In a preferred embodiment, -G₁- is a succinimide derivative, or an alkyl sulfone derivative which can contain one heteroatom such as oxygen or sulphur, or an ethyl amide derivative, or a 1,4-triazole derivative, or a multicycle derivative from a Diels-Alder reaction, or an aromatic phosphine derivative created by a Staudinger ligation, or a cysteine derivative coming from a Native Chemical Ligation.
   ∘ If c=0, then f₁, is an ether function, or a carbamate function.
   ∘ If c=1, then f₁, is an ether function, or a carbamate function, and f₄ is an amine function, or an amide function, or a carbamate function, or a thioether function, or an ether function, or a carbon-nitrogen covalent bond, or carbon-aromatic carbon covalent bond, or a carbon-carbon covalent bond if the crosslinking process is made by a Native Chemical Ligation (NCL)

In this embodiment, the cross-linked dextran polymer according to the invention is chosen among the dextran polymers of formula V. 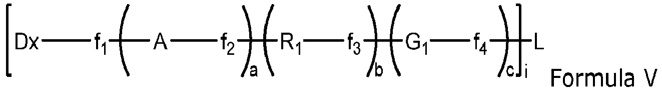 Wherein
- f₁, f₂, f₃, f₄, -A-, -R₁-, -G₁-are defined as above in Formula IV, and
- Dx- is a dextran moiety, which can be substituted by specific anionic groups in salified form, and optionally by alkyl carboxylate derivatives in salified form as previously defined.
- The integer i is the valence of the central-linker L, and the number of, identical or different, [Dx-f₁-(A-f₂)ₐ-(R₁-f₃)_{b}-(G₁-f₄)_{c}] radicals connected to L.
- The central-linker L is a polyether (PEG) derivative, which can be linear or branched.

In one embodiment L is linked to the same [Dx-f₁-(A-f₂)ₐ-(R₁-f₃)_{b}-(G₁-f₄)_{c}] radicals.

In one embodiment L is linked to different [Dx-f₁-(A-f₂)ₐ-(R₁-f₃)_{b}-(G₁-f₄)_{c}] radicals.

If a=0, then:
- In one embodiment, f₁ is an ether function.
- In another embodiment, f₁ is a carbamate function.

If a=1, then:
- In one embodiment, f₁ is an ether function.
- In another embodiment, f₁ is a carbamate function.

If a=0 and b=0, then:
- In one embodiment, f₁ is an ether function.
- In another embodiment, f₁ is a carbamate function.

If a=0 and b=1, then:
- In one embodiment, f₁ is an ether function.
- In another embodiment, f₁ is a carbamate function.

If a=b=c=0, then:
- In one embodiment, f₁ is an ether function.
- In another embodiment, f₁ is a carbamate function.

In one embodiment, the divalent radical -A- is a linear polyether (PEG) derivative chosen among the PEG of following formula:

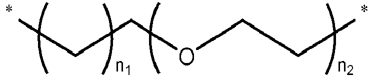

Wherein:
- n₁ is an integer equal to 0 or 1.
- n₂ is an integer comprised from 1 to 7 (1 ≤ n₁ ≤ 7).
- The ^{∗} represent the sites of f₁ and f₂.
- In a preferred embodiment, the ^{∗} represent the sites of f₁ and f₂, which are respectively ether and amide functions.

In another embodiment the divalent radical -A- is a 1-Alkyl-1,4-triazole derivative, or a 1-PEG-1,4-triazole derivative, chosen among the triazole derivative of following formula:

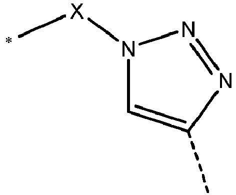

Wherein:
- X is either a linear ^{∗}-(CH₂)ₙ₁-^{∗} with n₁ an integer comprised from 1 to 7 (1 ≤ n₁ ≤ 7), branched, or cyclic alkyl derivative, or X is a PEG derivative.
- The ^{∗} represents the site of f₁ and the dotted bond represents f₂.

In another embodiment the divalent radical -A- is a 4-Alkyl-1,4-triazole derivative, or a 4-PEG-1,4-triazole derivative, chosen among the triazole derivative of following formula:

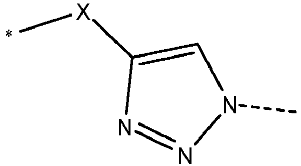

Wherein:
- X is either a linear ^{∗}-(CH₂)ₙ₁-^{∗} with n₁ an integer comprised from 1 to 7 (1 ≤ n₁ ≤ 7), branched, or cyclic alkyl derivative, or X is a PEG derivative.
- The ^{∗} represents the site of f₁ and the dotted bond represents f₂.

If a=1, then:
- In one embodiment, f₂ is an amide function.
- In another embodiment, f₂ is a carbon-nitrogen covalent bond.
- In another embodiment, f₂ is a carbon-aromatic carbon covalent bond.

In one embodiment, the divalent radical -R₁- is a linear alkyl derivative, according to the following formula:

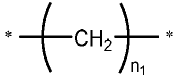

Wherein:
- n₁ is an integer comprised from 1 to 7 (1 ≤ n₁ ≤ 7).
- In one embodiment, if a=1, then the ^{∗} represent the sites of f₂ and f₃.
- In another embodiment, if a=0, the ^{∗} represent the sites of f₁ and f₃.

In another embodiment, the divalent radical -R₁- is a polyether (PEG) derivative, according to the following formula:

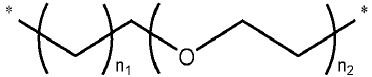

Wherein:
- n₁ is an integer equal to 0 or 1.
- n₂ is an integer comprised from 1 to 7 (1 ≤ n₂ ≤ 7).
- In one embodiment, if a=1, then the ^{∗} represent the sites of f₂ and f₃.
- In another embodiment, if a=0, the ^{∗} represent the sites of f₁ and f₃.

In another embodiment, if a=0, then the divalent radical -R₁- may be a branched alkyl, wherein at least one hydroxyl group is attached to the alkyl chain in β position from f₁, which is an ether function.

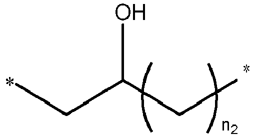

Wherein:
- n₂ is an integer comprised from 1 to 5 (1 ≤ n₂ ≤ 5).
- In another embodiment if a=0, the ^{∗} represent the sites of f₁ and f₃.

In a preferred embodiment, the divalent radical -R₁- is a linear alkyl derivative, according to the following formula:

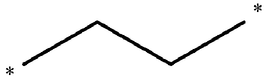

Wherein:
the ^{∗} represent the sites of f₂, which is an amide function, and f₃, which is an amide function.

In a preferred embodiment, the divalent radical -R₁- is a PEG derivative, according to the following formula:

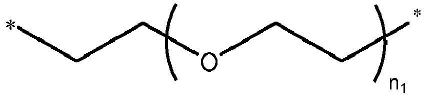

Wherein:
- n₁ is an integer comprised from 1 to 7 (1 ≤ n₁ ≤ 7).
- The ^{∗} represent the sites of f₂ and f₃, which are two amide functions.

If b=1, then:
- In one embodiment, f₃ is an amine function.
- In another embodiment, f₃ is an ether function.
- In another embodiment, f₃ is a thioether function.
- In another embodiment, f₃ is an amide function.
- In another embodiment, f₃ is a carbamate function.
- In another embodiment, f₃ is a carbon-nitrogen covalent bond.
- In another embodiment, f₃ is a carbon-aromatic carbon covalent bond.
- In another embodiment, if the crosslinking process is made by a Native Chemical Ligation (NCL), then f₃ is a carbon-carbon covalent bond.

The nature of radical G1 depends of the crosslinking process, below are described the different crosslinking process together with the G1 radicals.

In one embodiment, the crosslinking process is realized with a Michael addition with maleimide derivatives, or vinyl sulfone derivatives, or acrylamide derivatives.

In one embodiment, the divalent radical -G₁- is a succinimide derivative according to the following formula:

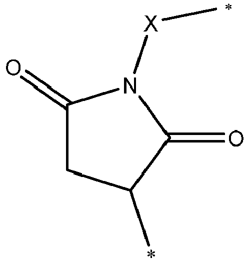

Wherein:
- X is either a linear ^{∗}-(CH₂)ₙ₁-^{∗} with n₁ an integer comprised from 1 to 7 (1 ≤ n₁ ≤ 7), branched, or cyclic alkyl derivative, or X is an aromatic, or X is a PEG derivative.
- In one embodiment if b=1, then the ^{∗} represent the sites of f₃ and f₄.
- In another embodiment if a=b=0, then the ^{∗} represent the sites of f₁ and f₄.
- In another embodiment if a=1 and b=0, then the ^{∗} represent the sites of f₂ and f4.
- In a preferred embodiment, X is an ethyl group and the ^{∗} represent the sites of f₂, which is an amide function, and f₄, which is an thioether function.

In another embodiment, the divalent radical -G₁- is a diethyl sulfone derivative according to the following formula:

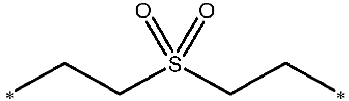

Wherein:
- In one embodiment if b=1, the ^{∗} represent the sites of f₃ and f₄.
- In another embodiment if a=b=0, the ^{∗} represent the sites of f₁ and f₄.
- In another embodiment if b=0, the ^{∗} represent the sites of f₂ and f₄.
- In a preferred embodiment, the ^{∗} represent the sites of f₃ and f₄, which are thioether functions.

In another embodiment, the divalent radical -G₁- is a sulfone derivative according to the following formula:

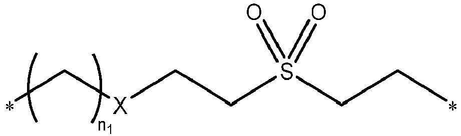

Wherein:
- n₁ is an integer comprised from 0 to 7 (0 ≤ n₁ ≤ 7).
- X is either an oxygen atom, or a sulphur atom, or a CH₂ group.
- In one embodiment if b= 1, the ^{∗} represent the sites of f₃ and f₄.
- In another embodiment if a=b=0, the ^{∗} represent the sites of f₁ and f₄.
- In another embodiment if a=1 and b=0, the ^{∗} represent the sites of f₂ and f₄.
- In preferred embodiment a=1, b=0, X is a sulphur atom, n₁=2, f₂ is an amide function, and f₄ is a thioether function.

In another embodiment, the divalent radical -G₁- is an acrylamide derivative according to the following formula:

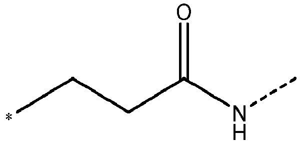

Wherein:
- In one embodiment, the ^{∗} represents the site of f₃, which is an amine function, or an ether function, or a thioether function, and the dotted bond represents f₄, which is a carbon-nitrogen covalent bond.
- In another embodiment, the dotted bond represents f₃, which is a carbon-nitrogen covalent bond, and the ^{∗} represents the site of f₄, which is an amine function, or an ether function, or a thioether function.

In one embodiment, the crosslinking process is realised with a 1,3-cycloaddition between alkyne and azide derivatives, known as 1,3-dipolar cycloaddition or Huisgen reaction.

In one embodiment, the divalent radical -G₁- is a 1,4-triazole derivative according to the following formula:

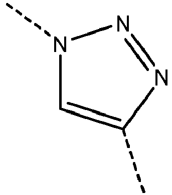

Wherein the two dotted bonds represent f₃ and f₄ which are covalent bonds or chemical functions defined previously and after.

In one embodiment, the crosslinking process is realised with a 1,3-cycloaddition between strain alkyne and azide derivatives, known as Strain-promoted azide-alkyne cycloaddition, or SPAAC.

In one embodiment, the divalent radical -G₁- is a triazole derivative according to the following formula

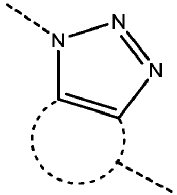

Wherein:
- The dotted circle represents a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine.
- The two dotted bonds represent f₃ and f₄, which are covalent bonds or chemical functions defined previously and after.
- In another embodiment if a=b=0, the two dotted bonds represent f₁ and f₄.
- In another embodiment if a=1 and b=0, the two dotted bonds represent f₂ and f4.
- In a preferred embodiment, the dotted bonds represent f₂, which is an amide function, and f₄, which is a carbon-nitrogen covalent bond.

In another embodiment, the divalent radical -G₁- is a triazole derivative according to the following formula:

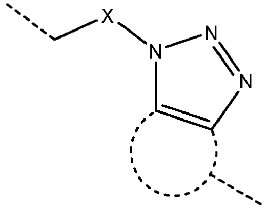

Wherein:
- The dotted circle represents a cyclooctene derivative, coming from strained cyclooctyne, which can contain one heteroatom such as nitrogen, oxygen, or sulphur, and is optionally functionalised by linear, branched, or cyclic alkyl derivatives comprising between 2 to 20 carbon atoms, or by aromatics derivatives, or by heteroatoms such as nitrogen, oxygen, or sulphur, or halogens, especially fluorine.
- X is either a linear ^{∗}-(CH₂)ₙ₁-^{∗} with n₁ an integer comprised from 1 to 7 (1 ≤ n₁ ≤ 7), branched, or cyclic alkyl derivative, or X is a PEG derivative.
- The two dotted bonds represent f₃ and f₄, which are covalent bonds or chemical functions defined previously and after.
- In another embodiment if a=b=0, the ^{∗} represent the sites of f₁, and the dotted bond represents f₄.
- In another embodiment if a=1 and b=0, the ^{∗} represent the sites of f₁, and the dotted bond represents f₄.
- In a preferred embodiment, X is a PEG derivative, the dotted bonds represent f₂ and f₄, which are amide functions

In a preferred embodiment, the divalent radical -G₁- is a triazole derivative according to the following formula: 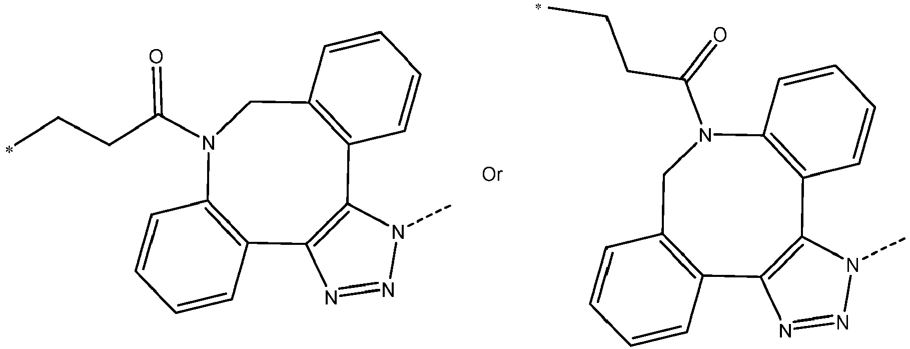 Wherein: the ^{∗} represents the site of f₂, which is an amide function, and the dotted bond represents f₄, which is a carbon-nitrogen bond.

In another preferred embodiment, the divalent radical -G₁- is a triazole derivative according to the following formula:

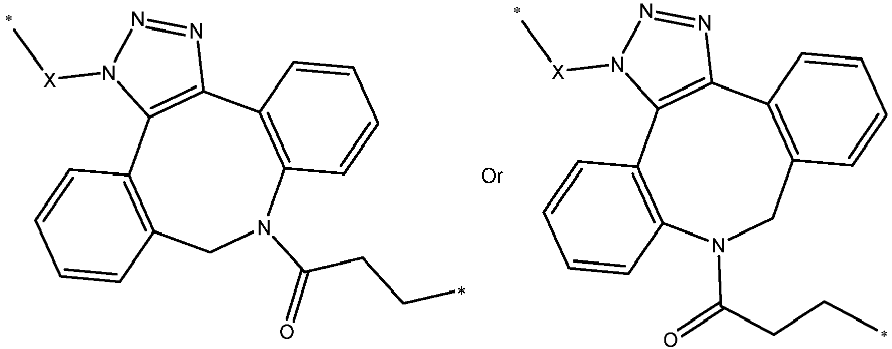

Wherein:
- X is a PEG derivative.
- The ^{∗} represent the sites of f₂ and f₄, which are amide functions.

In another embodiment, the divalent radical -G₁- is a triazole derivative according to the following formula:

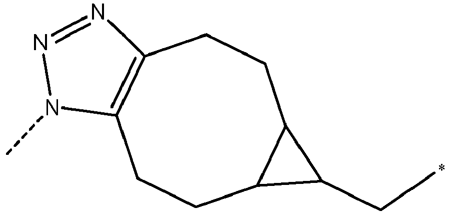

Wherein: the ^{∗} represents the site of f₃, and the dotted bond represents f₄.

In another embodiment, the divalent radical -G₁- is a triazole derivative according to the following formula:

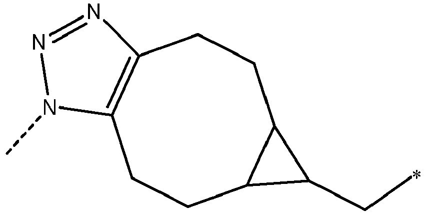

Wherein, the dotted bond represents f₃, and the ^{∗} represents the site of f₄.

In one embodiment, the crosslinking process is realized with a Diels-Alder cycloaddition between maleimide and furane derivatives.

In one embodiment, the divalent radical -G₁- is a multiple cycle derivative, composed of one succinimide moiety, according to the following formula:

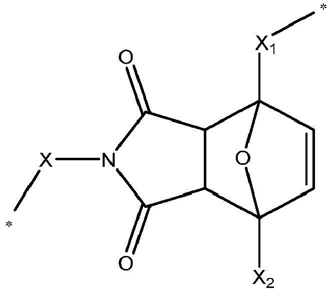

Wherein:
- X is either a linear ^{∗}-(CH₂)ₙ₁-^{∗} with n₁ an integer comprised from 1 to 7 (1 ≤ n₁ ≤ 7), branched, or cyclic alkyl derivative, or X is a PEG derivative.
- X₁ is either a linear ^{∗}-(CH₂)ₙ₁-^{∗} with n₁ an integer comprised from 0 to 7 (0 ≤ n₁ ≤ 7), branched, or cyclic alkyl derivative.
- X₂ is either -H or -Me.
- In one embodiment if a=b=1, the ^{∗} represent the sites of f₃ and f₄.
- In another embodiment if a=b=0, the ^{∗} represent the sites of f₁ and f₄.
- In another embodiment if a=1 and b=0, the ^{∗} represent the sites of f₂ and f₄.

In one embodiment, the crosslinking process is realised with an inverse electron-demand Diels-Alder reaction or IEDDA, between tetrazine and norbornene derivatives.

In one embodiment, the divalent radical -G₁- is a multiple cycle derivative, composed of one pyridazine moiety, according to the following formula:

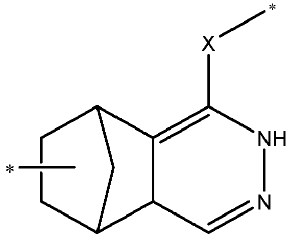

Wherein:
- X is either a linear ^{∗}-(CH₂)ₙ₁-^{∗} with n₁ an integer comprised from 1 to 7 (1 ≤ n₁ ≤ 7), branched, or cyclic alkyl derivative, or X is an aromatic derivative, or X is a PEG derivative.
- In one embodiment if a=b=1, the ^{∗} represent the sites of f₃ and f₄.
- In another embodiment if a=b=0, the ^{∗} represent the sites of f₁ and f₄.
- In another embodiment if a=1 and b=0, the ^{∗} represent the sites of f₂ and f₄.

In another embodiment, the divalent radical -G₁- is a multiple cycle derivative, composed of one pyridazine moiety, according to the following formula:

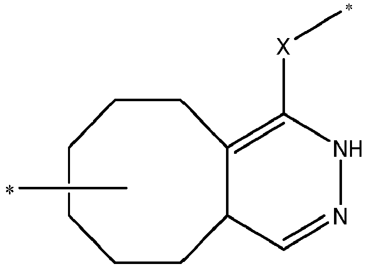

Wherein:
- X is either a linear ^{∗}-(CH₂)ₙ₁-^{∗} with n₁ an integer comprised from 1 to 7 (1 ≤ n₁ ≤ 7), branched, or cyclic alkyl derivative, or X is an aromatic derivative, or X is a PEG derivative.
- In one embodiment if a=b=1, the ^{∗} represent the sites of f₃ and f₄.
- In another embodiment if a=b=0, the ^{∗} represent the sites of f₁ and f₄.
- In another embodiment if a=1 and b=0, the ^{∗} represent the sites of f₂ and f₄.

In one embodiment, the crosslinking process is realised with a Staudinger ligation, between an aromatic phosphine and an azide derivative.

In one embodiment, the divalent radical -G₁- is an aromatic derivative, according to the following formula:

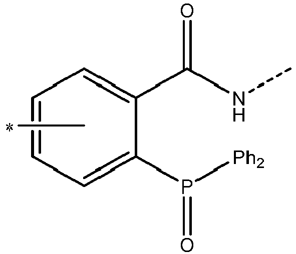

Wherein:
- In one embodiment if a=b=1, the ^{∗} represent the sites of f₃ and f₄.
- In another embodiment if a=b=0, the ^{∗} represent the sites of f₁ and f₄.
- In another embodiment if a= 1 and b=0, the ^{∗} represent the sites of f₂ and f₄.

In one embodiment, the crosslinking process is realised with a Native Chemical Ligation (NCL), between thioester and N-terminal cysteine derivatives.

In one embodiment, the divalent radical -G₁- can be formalised according to the following formula:

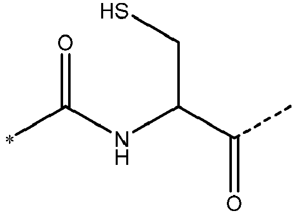

Wherein:
- The dotted line represents a carbon-nitrogen covalent bond.
- In one embodiment if a=b=1, the ^{∗} and the dotted line represent the sites of f₃ and f₄.
- In another embodiment if a=b=0, the ^{∗} and the dotted line represent the sites of f₁ and f₄.
- In another embodiment if a=1 and b=0, the ^{∗} and the dotted line represent the sites of f₂ and f₄.

If c=1, then:
- In one embodiment, f₄ is an amine function.
- In another embodiment, f₄ is an ether function.
- In another embodiment, f₄ is a thioether function.
- In another embodiment, f₄ is an amide function.
- In another embodiment, f₄ is a carbamate function
- In another embodiment, f₄ is a carbon-nitrogen covalent bond.
- In another embodiment, f₄ is a carbon-aromatic carbon covalent bond.
- In another embodiment, if the crosslinking process is made by a Native Chemical Ligation (NCL), then f₄ is a carbon-carbon covalent bond.

The invention also concerns the dextran polymers of formula VIII before the crosslinking reaction.

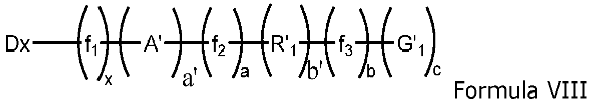

Wherein
- f₁, f₂, f₃, Dx are defined as above if none of a, a', b and b' are equal to 0,
- and
- x equal 0 or 1.
- if a, a', b and b' are equal to 0, x is equal to 0 and Dx is a dextran polymer backbone according to formula III, wherein R is chosen among -H or a anionic group of formula II,
- if one of b' and c is not equal to 0 -A'- is -A- as defined above,
- if b', b and c are equal to 0, a is equal to 0 and A' is the precursor of A before the crosslinking reaction.
- if c is not equal to 0, R'₁ is R₁ as defined above and G'₁ is the precursor of G₁.
- if c is equal to 0, b is equal to 0 and R'₁ is the precursor of R₁ before the crosslinking , reaction.

In an embodiment A' is an alkyl carboxylate derivative or a poly(oxyethylene)carboxylate derivative, or an alkyl azide derivative, or a poly(oxyethylene)azide derivative, or a propargyl derivative, or a poly(oxyethylene)propargyl derivative, or a 2-hydroxyalkyl carboxylate, or a 2-hydroxyalkylamine.

A' as carboxylate derivatives can be formalized with the following formulas:

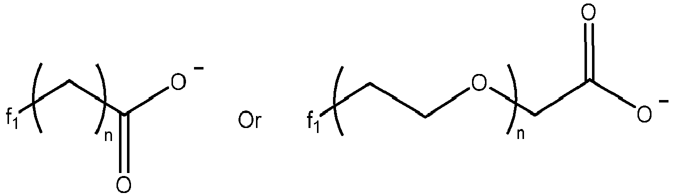

A' as azide derivatives can be formalized with the following formulas:

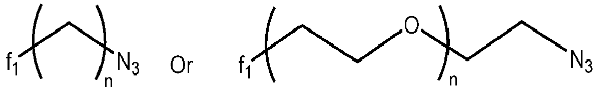

A' as propargyl derivatives can be formalized with the following formulas:

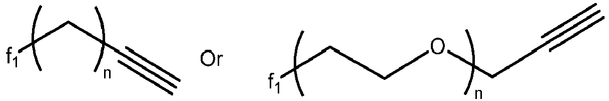

A' as a 2-hydroxyalkyl carboxylate derivative can be formalized with the following formula:

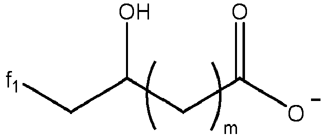

A' as a 2-hydroxyalkylamine derivatives can be formalized with the following formula:

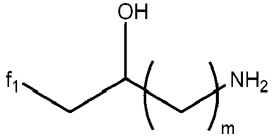

Wherein:
- n is an integer comprised from 1 to 7 (1 ≤ n ≤ 7)
- m is an integer comprised from 1 to 5 (1 ≤ m ≤ 5)
- f₁ is defined as previously

In an embodiment, if a=1, R'1 is an alkyl radical or a poly(oxyethylene) radical bearing a terminal amine, or a terminal hydroxyl, or a terminal thiol, or a terminal carboxylate, or a terminal azide, or a terminal alkyne.

R'1 can be formalized as follows:

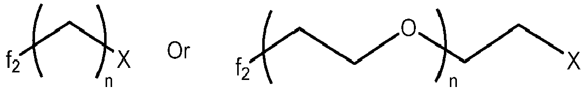

R'₁ as carboxylate derivatives can be formalized with the following formulas:

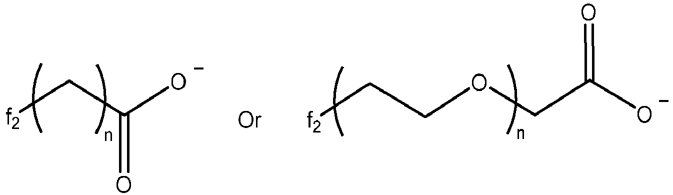

R'₁ as azide derivatives can be formalized with the following formulas:

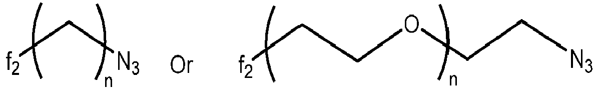

Wherein:
- n is an integer comprised from 1 to 7 (1 ≤ n ≤ 7)
- X= -NH₂, or -OH, or -SH
- f₂ is defined as previously

Or, in another embodiment, if a=0, then R'₁ is a branched alkyl, wherein at least one hydroxyl group is attached to the alkyl chain in β position from f₁, and having a terminal hydroxyl, or a terminal thiol, or a terminal azide, or a terminal alkyne.

R'₁ can be formalized as follows:

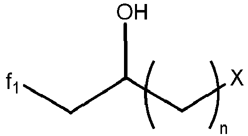

Wherein:
- n is an integer comprised from 1 to 5 (1 ≤ n ≤ 5)
- X= -NH₂, or -OH, or -SH, or -N₃, or -C=CH
- f₁ is defined as previously

Or, in another R'₁ is a propargyl derivatives, and can be formalized with the following formulas:

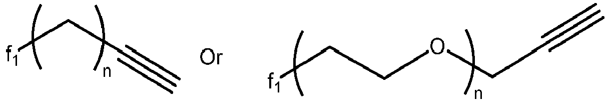

Wherein:
- n is an integer comprised from 1 to 7 (1 ≤ n ≤ 7)
- f₁ is defined as previously

In an embodiment G'1 is a maleimide derivative, or a vinylsulfone derivative, or a strained cyclooctyne derivative, or an azide derivative, or propargyl derivative, or a furane derivative, or an acrylamide derivative, or a norbornene derivative, or a trans-cyclooctene derivative, or a tetrazine derivative, or an aromatic phosphine, or a cysteine, or a thioester, or a thiol derivative, or an amine derivative, or a hydroxyl derivative.

G'1 as a thiol, an amine or a hydroxyl derivative can be formalized as follow:

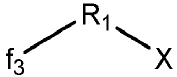

G'₁ as a maleimide can be formalized as follows:

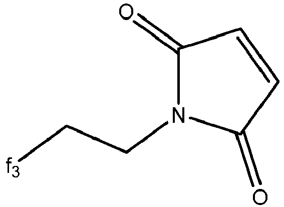

G'₁ as a vinylsulfone can be formalized as follows:

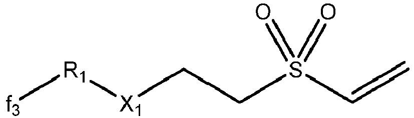

G'₁ as a strained cyclooctyne can be formalized as follows:

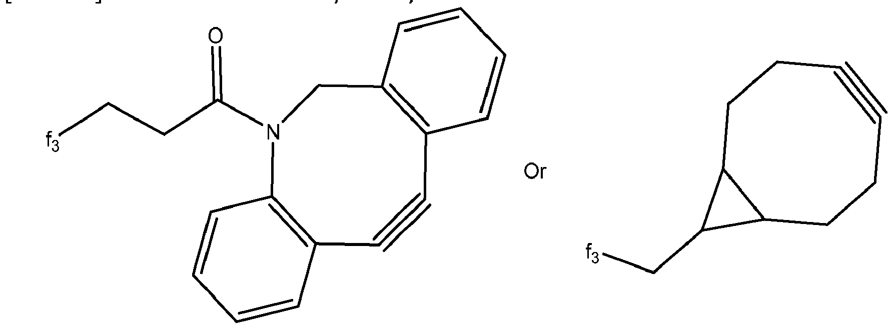

G'₁ as azide derivatives can be formalized with the following formula:

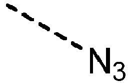

G'₁ as propargyl derivatives can be formalized with the following formula:

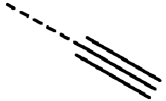

G'₁ as furane derivatives can be formalized with the following formula:

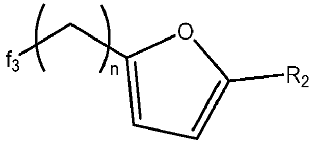

G'₁ as acrylamide derivatives can be formalized with the following formula:

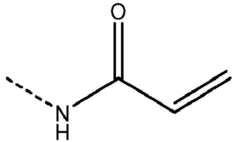

G'₁ as norbornene derivatives can be formalized with the following formula:

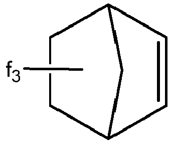

G'₁ as trans-cyclooctene derivatives can be formalized with the following formula:

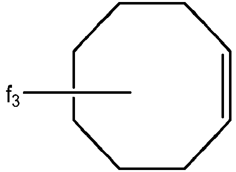

G'₁ as tetrazine derivatives can be formalized with the following formula:

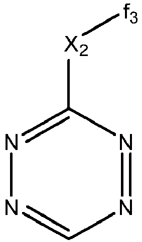

G'₁ as aromatic phosphine derivative can be formalized with the following formula:

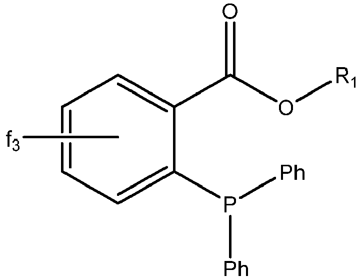

G'₁ as cysteine derivatives can be formalized with the following formula:

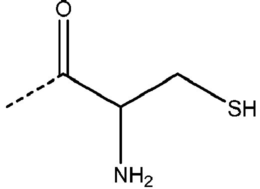

G'₁ as thioester derivatives can be formalized with the following formula:

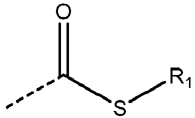

Wherein:
- X= -NH₂, or -OH, or -SH
- X₁= -O-, or -S-
- n is an integer equal to 0 or 1
- R₁= Alkyl
- X₂= -CH₂-, or aromatic
- R₂= -H, or -CH₃
- f₃ is defined as previously
- The dotted bonds represent f₃, which is a carbon-nitrogen covalent bond, or a carbon-carbon covalent bond

The invention also concerns a hydrogel comprising the cross-linked dextran polymer according to the invention.

In an embodiment the hydrogel is transparent.

By "transparent" is meant that in conditions disclosed in Example C21 of application PCT/EP2022/050466 for visual inspection an observer considered the sample transparent compared to the standard 2 (6 NTU) and/or the UV absorbance of the hydrogel as measured in Example C21 of application PCT/EP2022/050466 is lower than 0.06 (Absorbance Units).

In an embodiment the hydrogel is visually transparent and has a UV absorbance < 0.06 (Abs. Units).

In an embodiment the hydrogel according to the invention is characterized in that Tan δ is lower than 1.

In the present specification, Tan δ is the ratio of the storage modulus (also called elastic modulus) G' to the loss modulus G" (Tan δ = G'/G").

In an embodiment the hydrogel according to the invention is characterized in that Tan δ is less than or equal to 0.5.

In an embodiment the hydrogel according to the invention is characterized in that Tan δ is less than or equal to 0.1.

In an embodiment the hydrogel according to the invention is characterized in that Tan δ is less than or equal to 0.05.

In an embodiment the hydrogel according to the invention is characterized in that Tan δ is less than or equal to 0.01.

In an embodiment the hydrogel according to the invention is characterized in that after swelling in water the cross-linked dextran polymer concentration is comprised from 0.01 to 0.2 g/g.

In an embodiment the hydrogel according to the invention is characterized in that after swelling in water the cross-linked dextran polymer concentration is comprised from 0.03 to 0.1 g/g.

In an embodiment the hydrogel according to the invention is characterized in that after swelling in water the cross-linked dextran polymer concentration is comprised from 0.05 to 0.1 g/g.

In an embodiment, the hydrogel is translucid.

In another embodiment the hydrogel is transparent.

In an embodiment, the hydrogel has a Young modulus comprised between 1 to 200 kPa.

In an embodiment, the hydrogel has a Young modulus comprised between 5 to 200 kPa.

In an embodiment, the hydrogel has a Young modulus comprised between 20 to 200 kPa.

In an embodiment, the hydrogel has a Young modulus comprised between 30 to 200 kPa.

In an embodiment, the hydrogel has a Young modulus comprised between 50 to 200 kPa.

In an embodiment, the hydrogel has a Young modulus comprised between 30 to 180 kPa.

In an embodiment, the hydrogel has a Young modulus comprised between 50 to 150 kPa.

In an embodiment, the hydrogel has a Young modulus comprised between 5 to 100 kPa.

In an embodiment, the hydrogel has a Young modulus comprised between 10 to 90 kPa.

In an embodiment, the hydrogel has a Young modulus comprised between 10 to 75 kPa.

In an embodiment, the hydrogel has a G' comprised from 0.5 to 70 kPa.

In an embodiment the hydrogel has a compression deformation at break of more than or equal to 10 %.

In an embodiment the hydrogel has a compression deformation at break of more than or equal to 15 %.

In an embodiment the hydrogel has a compression deformation at break of more than or equal to 20 %.

In an embodiment the hydrogel has a compression deformation at break of more than or equal to 25 %.

In an embodiment the hydrogel has a compression deformation at break of more than or equal to 30 %.

In an embodiment the hydrogel has a compression deformation at break of more than or equal to 35 %.

In an embodiment the hydrogel has a compression deformation at break of more than or equal to 40 %.

In an embodiment the hydrogel has a compression deformation at break of more than or equal to 45 %.

In an embodiment the hydrogel has a compression deformation at break of more than or equal to 50 %.

In an embodiment the hydrogel has a compression deformation at break of more than or equal to 55 %.

In an embodiment the hydrogel has a compression deformation at break of more than or equal to 60 %.

In an embodiment the hydrogel has a traction deformation at break of more than or equal to 10 %.

In an embodiment the hydrogel has a traction deformation at break of more than or equal to 15 %.

In an embodiment the hydrogel has a traction deformation at break of more than or equal to 20 %.

In an embodiment the hydrogel has a traction deformation at break of more than or equal to 25 %.

In an embodiment the hydrogel has a traction deformation at break of more than or equal to 30 %.

In an embodiment the hydrogel has a traction deformation at break of more than or equal to 35 %.

In an embodiment the hydrogel has a traction deformation at break of more than or equal to 40 %.

In an embodiment the hydrogel has a swelling ratio of more than 0.7.

In an embodiment the hydrogel has a swelling ratio of more than 0.8.

In an embodiment the hydrogel has a swelling ratio of more than 0.9.

In an embodiment the hydrogel has a swelling ratio of more than 1.

In an embodiment the hydrogel has a swelling ratio of more than 1.1.

In an embodiment the hydrogel has a swelling ratio of more than or equal to 1.2.

In an embodiment the hydrogel has a swelling ratio of more than or equal to 1.3.

In an embodiment the hydrogel has a swelling ratio of more than or equal to 1.4.

In an embodiment the hydrogel has a swelling ratio of more than or equal to 1.5.

In an embodiment the hydrogel has a swelling ratio of more than or equal to 1.6.

In an embodiment the hydrogel has a swelling ratio of less than or equal to 5.

In an embodiment the hydrogel has a swelling ratio of less than or equal to 4.

In an embodiment the hydrogel has a swelling ratio of less than or equal to 3.

In an embodiment the hydrogel has a swelling ratio of less than or equal to 2.8.

In an embodiment the hydrogel has a swelling ratio of less than or equal to 2.5.

In an embodiment the hydrogel has a swelling ratio of less than or equal to 2.3.

In an embodiment the hydrogel has a water content of at least 80 wt%.

In an embodiment the hydrogel has a water content of at least 85 wt%.

In an embodiment the hydrogel has a water content of at least 90 wt%.

In an embodiment the hydrogel has a water content of at least 97 wt%.

In an embodiment the hydrogel has a water content of at least 96 wt%.

In an embodiment the hydrogel has a water content of at least 95 wt%.

In an embodiment the hydrogel has a water content of at least 94 wt%.

In an embodiment the hydrogel has a water content of at least 93 wt%.

In an embodiment the hydrogel has a water content of at most 99 wt%.

In an embodiment the hydrogel has a water content of at most 98 wt%.

In an embodiment the hydrogel according to the invention is characterized in that it further comprises an API.

In an embodiment, the cross-linked dextran polymer according to the invention is a dextran polymer bearing anionic groups of formula II wherein the at least divalent radical L(-)ᵢ is covalently bound to the dextran polymer backbone with i -W-radicals, wherein i is 2, 4 or 8.

In an embodiment, the cross-linked dextran polymer according to the invention is a dextran polymer bearing anionic groups of formula II wherein the at least divalent radical L(-)ᵢ is covalently bound to the dextran polymer backbone with i -Wradicals, wherein, i is 2.

In an embodiment, the cross-linked dextran polymer according to the invention is a dextran polymer bearing anionic groups of formula II wherein the at least divalent radical L(-)ᵢ is covalently bound to the dextran polymer backbone with i -W-radicals, wherein, i is 4.

In an embodiment, the cross-linked dextran polymer according to the invention is a dextran polymer bearing anionic groups of formula II wherein the at least divalent radical L(-)ᵢ is covalently bound to the dextran polymer backbone with i -W- - radicals, wherein, i is 8.

The cross-linking step is a gelation step that leads to the formation of a hydrogel according to the invention.

The hydrogel formation kinetic is function of the temperature and could be modulated by the reactant's concentrations, pH and temperature.

In an embodiment the time to obtain a hydrogel according to the invention is comprised from 1 minute to 6 hours.

In an embodiment the cross-linking step is carried out for 1 hour.

In an embodiment the temperature of the cross-linking step is comprised from 4°C to room temperature (20-25°C) and could vary between the step of mixing and the step of gelation and moulding.

In an embodiment the mixing is performed at 4°C and the gelation is carried out at room temperature (20-25°C) for 1 hour.

In an embodiment the mixing is performed at room temperature (20-25°C).

In an embodiment the mixing is performed at 4°C or room temperature (20-25°C) and the gelation is carried out at room temperature (20-25°C) for 1 hour.

In an embodiment, the gelation is carried out at 37 °C.

In an embodiment, after cross-linking or gelation, the hydrogel is swelled in a buffer solution, the pH of the buffer solution is comprised from 5 to 8, preferably from 6 to 8 and more preferably from 6.8 to 7.5.

In an embodiment, the buffer solution is a PBS solution at pH 7.4.

In an embodiment, the buffer solution is a Tris solution at pH 7.4.

In an embodiment, the buffer solution is a Tris solution at pH 8.

In an embodiment the swelling allows the hydrogel mass being increased by 1, 2, 3 or 4 compared to its initial mass.

The invention also concerns a process to synthetize a cross-linked dextran polymer according to the invention, into the form of a hydrogel, comprising the steps of:
a) preparation of a sterile solution comprising a dextran bearing anionic groups of formula II and at least two precursors of -W-
b) preparation of a sterile solution of a precursor of L(-)ᵢ
c) addition of the sterile solution obtained from step b) to the solution obtained from step a),
d) the addition being directly done in a mould or the solutions are introduced into a mould after being mixed,
e) cross-linking and gelation, for example at room temperature (20-25°C) or at 37°C,
f) unmoulding and swelling to obtain an hydrogel.

In an embodiment steps c) and d) are done simultaneously.

In an embodiment, the swelling is done into a PBS solution at pH 7,4.

Dextrans bearing anionic groups of formula II are prepared by grafting or substitution on the hydroxyl groups borne by the dextrans. In an embodiment the dextrans bearing anionic groups of formula II are prepared by grafting or substituting the carboxymethyl groups borne by the carboxymethyl dextrans.

In an embodiment of the process according to the invention an active pharmaceutical ingredient (API) is entrapped into the hydrogel.

The invention also concerns a therapeutic use of the hydrogel according to the invention as a therapeutic implant to administer the API to a mammal.

The invention also concerns a process to prepare a hydrogel comprising an API comprising the steps of:
a) preparation of a sterile solution comprising a dextran a dextran bearing anionic groups of formula II and at least two precursors of -W-,
b) preparation of a sterile solution of a precursor of L(-)ᵢ,
c) preparation of a suspension of an API,
d) mixing the API suspension obtained from step c) and the solution obtained from the step b) or a),
e) addition of the sterile solution obtained from step a) or b) which is not used in step d) to the suspension obtained from step d),
f) the addition of step e) being either done directly in a mould or the solutions are introduced into a mould after being mixed,
g) cross-linking and gelation reaction at room temperature (20-25°C),
h) unmoulding and swelling to obtain an hydrogel comprising an API.

The invention also concerns a process to prepare a hydrogel comprising an API comprising the steps of:
a) preparation of a sterile solution comprising a dextran a dextran bearing anionic groups of formula II and at least two precursors of -W-,
b) preparation of a sterile solution of a precursor of L(-)ᵢ,
c) preparation of a suspension of an API,
d) mixing the API suspension obtained from step c) and the solution obtained from the step b) or a),
e) addition of the sterile solution obtained from step a) or b) which is not used in step d) to the suspension obtained from step d),
f) the addition of step e) being either done directly in the barrel of a syringe or the solution / suspension are introduced into the barrel of a syringe after being mixed,
g) injection of the mixture from step f).
h) cross-linking and gelation reaction at room temperature (20-25°C),
i) unmoulding and swelling to obtain an hydrogel comprising an API.

In an embodiment, the swelling is done into a PBS solution at pH 7.4.

The invention also concerns an implantable device comprising at least a hydrogel according to the invention and obtained according to the process of the invention.

The invention also concerns an implant consisting of the hydrogel according to the invention used for drug delivery.

The invention also concerns an implant comprising the hydrogel according to the invention used for drug delivery.

The invention also concerns an implant comprising the hydrogel according to the invention and an API used for drug delivery.

In an embodiment, at least 50 % of the surface the hydrogel is directly in contact with the medium in which it is implanted.

In an embodiment, at least 75 % of the surface the hydrogel is directly in contact with the medium in which it is implanted.

In an embodiment, at least 90 % of the surface the hydrogel is directly in contact with the medium in which it is implanted.

In an embodiment, at least 95 % of the surface the hydrogel is directly in contact with the medium in which it is implanted.

In an embodiment, 99 % of the surface the hydrogel is directly in contact with the medium in which it is implanted.

In an embodiment, at least 50 % of the surface the hydrogel is directly in contact with the exterior of the device or implant.

By « directly in contact with the exterior » means there is no separation between the hydrogel and the exterior, for example no wall made of a non-hydrogel material between the hydrogel and the exterior of the device or implant.

In an embodiment, at least 75 % of the surface the hydrogel is directly in contact with the exterior of the device or implant.

In an embodiment, at least 90 % of the surface the hydrogel is directly in contact with the exterior of the device or implant.

In an embodiment, at least 95 % of the surface the hydrogel is directly in contact with the exterior of the device or implant.

In an embodiment, at least 99 % of the surface the hydrogel is directly in contact with the exterior of the device or implant.

In an embodiment, 100 % of the surface the hydrogel is directly in contact with the exterior of the device or implant.

The API are entrapped into the maze of cross-linked dextran hydrogel.

In this specification the word "entrapped" is equivalent to "encapsulated" or "encapsulation".

The hydrogel matrix allows passage of small molecules e.g. API, API being entrapped into the hydrogel.

Typically, API are hormone and peptide drugs chosen amongst PTH protein, insulin and coagulation factors.

In an embodiment, this mesh size is less than 1 µm.

In another embodiment it is less that 100 nanometers, preferably less than 10 nanometers, and more preferably around 5 nanometers.

In an embodiment the invention concerns an implant comprising a ring, a net, the hydrogel according to the invention and an API.

The ring and net structure allow the hydrogel to be easily manipulated, a good resistance to manipulation, including for implantation. This is also true even with a hydrogel having a larger mesh size (for example with a lower DS of-W-and lower concentrations of reactive groups during crosslinking).

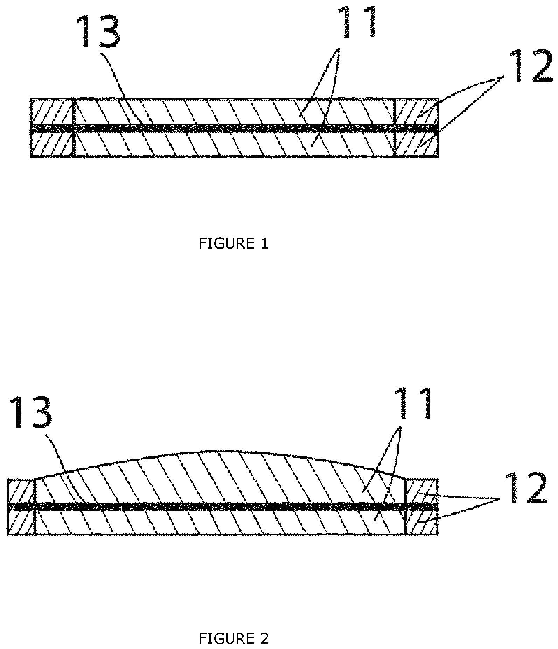

Figure 1 represents an implant (1) comprising a hydrogel (11) comprising an API (not represented), a ring (12) and a net (13). Upper part of the ring, lower part of the ring and net can be glued together (not represented).

Figure 2 represents an implant (1) comprising a hydrogel (11) comprising an API (not represented), a ring (12) and a net (13), where the hydrogel is concave.

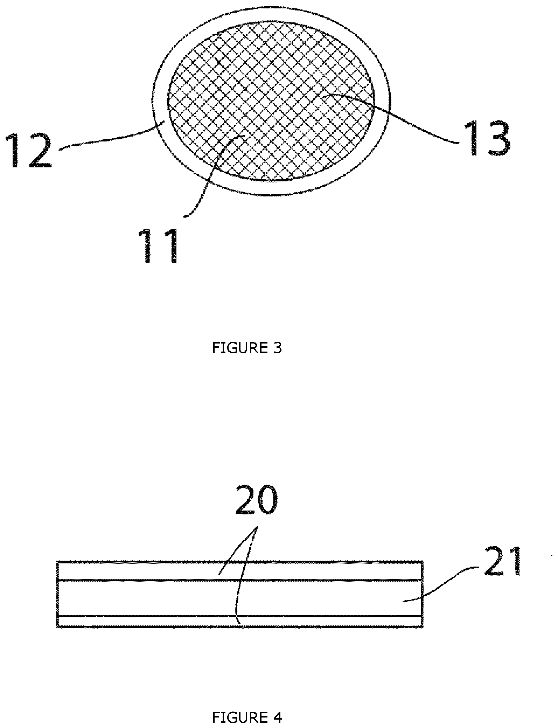

Figure 3 is a top view of an implant (1) comprising a hydrogel (11) comprising an API (not represented), a ring (12) and a net (13).

Figure 4 represents an implant (1) comprising a hydrogel without API(20) sandwiching an hydrogel comprising an API (21). The upper part of 20 being optional.

In an embodiment the ring has an internal diameter of 10 to 100 mm.

In an embodiment the ring has an internal diameter of 15 to 50 mm.

In an embodiment the ring has a diameter of 0.5 to 5 mm

In an embodiment the ring has a diameter of 0.5 to 10 mm.

In an embodiment the ring has a diameter of 0.5 to 5 mm.

In an embodiment the ring has a total (lower plus upper part and glue) thickness of 100 to 3000 µm.

In an embodiment the ring has a total (lower plus upper part and glue) thickness of 150 to 2000 µm.

In an embodiment the ring has a total thickness of 200 to 5000 µm.

In an embodiment the ring has a total thickness of 500 to 3000 µm.

In an embodiment the ring has a rectangular, square or round section.

In an embodiment the ring material is a bioinert material.

In an embodiment, the ring material is a biocompatible elastomer.

In an embodiment the ring material is chosen from the group consisting of silicone, in particular PDMS, polyurethanes, polyether, polyether polyester copolymers and polypropylene oxide.

In an embodiment the ring material is silicone.

In an embodiment the ring material is PDMS.

In an embodiment the net is non-biodegradable.

In an embodiment the net is biocompatible.

In an embodiment the net is non absorbable.

In an embodiment the net is a surgical mesh.

In an embodiment the filament material of the net material is chosen among the group consisting of Polypropylene, Polyethylene, polyester, in particular PET, PTFE, PVDF (polyvinylidene fluoride) and ePVDF (extended PVDF).

In an embodiment the filament material of the net is chosen among the group consisting of Polypropylene, polyester, in particular PET, PTFE and PVDF (polyvinylidene fluoride).

In an embodiment the filament material of the net is chosen among the group consisting of Polypropylene and polyester, in particular PET.

In an embodiment the filament material of the net is chosen among the group consisting of Polypropylene.

In an embodiment the filament material of the net is chosen among the group consisting of is polyester, in particular PET.

In an embodiment the net has a thickness ranging from 50 to 500 µm.

In an embodiment the m net has a thickness ranging from 100 to 300 µm.

In an embodiment, the filament diameter is ranging from 0.08 to 0.2 mm.

In an embodiment the pore size is ranging from 0.4 to 4 mm.

In an embodiment the pore size is ranging from 0.6 to 2 mm.

In an embodiment fabric of the net is chosen from the group consisting of knitted fabric, warp knitted fabric, woven fabric, non-woven fabric.

In an embodiment fabric of the net is chosen from the group consisting of warp knitted fabric, in particular multi-filament.

In an embodiment the net is treated in order to increase the hydrophilicity.

In an embodiment the net is treated with a base, in particular on polyester, more particularly on PET.

In an embodiment this treatment is functionalisation of the surface from reactive function, such as -OH, -COOH, and reactive molecules or polymer.

The grafted polymer could thus expose reactive functions for a further reaction with the hydrogel or a precursor of hydrogel, such as a thiol function.

In an embodiment this treatment is done by adsorption of synthetic polymers, such as poloxamers or polyvinyl pyrrolidone (PVP) or of natural polymers, such as collagen, or of surfactants after a chemical or physical treatment.

In an embodiment the net remains below the exterior end of the ring.

In an embodiment the net is not in contact with the exterior of the implant comprising a ring, a net and the hydrogel.

In an embodiment the glue is biocompatible.

In an embodiment the glue is a biocompatible silicone glue, such as Silbione MED ADH 4200 supplied by Elkem.

In an embodiment the glue remains below the exterior end of the ring.

For example, a warp knit Polyester surgical net fabric type PETKM3002 (1x0.9mm pore size) supplied by SurgicalNet^{™} was treated in NaOH 1M for 5 hours at 70°C and rinsed with deionized water and ethanol 96%. The treatment led to an increased hydrophilicity of the net fabric leading to an improved wetting with aqueous solutions.

For example, a ring net construct may be obtained following the process below:
- Biocompatible PDMS sheets supplied by Grace Biolabs or Interstate Speciality Product is cut in a form of a square incorporating a circular empty disc using a stainless steel punch,
- A part of the treated polyester surgical net described is introduced in between two square PDMS pieces: The two PDMS pieces and the surgical net are glued together with biocompatible silicone glue (Silbione MED ADH 4200 supplied by Elkem). The circular empty discs are aligned, and the surgical net is kept tense during gluing,
- then, the square construct is cut with a strainless steel punch to obtain the final object constituted by two PDMS rings sandwiching a surgical net and glued together, and
- The pieces are washed with a solution of poloxamer F127 at 1% and rinsed with water before steam sterilization.

In an embodiment, the implant can be obtained by the following process:
- Hydrogel compositions are incorporated in the Ring Mesh constructs. The concentrated polymer solutions are mixed with a pipette and a controlled volume of the mixture is introduced in a ring net construct adhering to a glass slide,
- Crosslinking leading to gelation is carried out. Then the Ring Mesh + Hydrogel composition is introduced in a Tris 150 mM /NaCl 30 mM / Cystein 10 mM solution at pH 8 or in PBS at pH 7.4,
- The hydrogel was rinsed with PBS solution without cysteine and further immersed in the PBS solution overnight at 37°C. The hydrogel piece was then stored in PBS solution at 4°C until being used.

Hydrogel/Ring Mesh implants can then be easily manipulated with tweezers and are foldable for the need of surgical implantation. Moreover, it is possible to fix the ring with sutures.

The hydrogel volume can be adjusted with the internal diameter and the thickness or the ring net construct. For the same ring net construct the hydrogel volume can be adjusted to control the convexity / concavity of the hydrogel above the ring level.

In an embodiment, the hydrogel comprises a first layer of the hydrogel wich does not comprise API and a second layer of the hydrogel which comprises API.

Such a structure may be obtained with a process as disclosed in this application, but with two steps of adding hydrogel precursors first step is adding hydrogel precursors without API as a first layer, and the second step is adding the hydrogel precursor with API while the gelation of the first step is not finished, in particular at a time corresponding to 5 to 25 % of the gelation time of the first hydrogel, as a second layer.

### EXAMPLES

### Part A - CHEMISTRY

### Example A1: Synthesis of substituted dextran

**Table 1: List of synthesized polysaccharide**

| **Polysaccharide** | **Structure** | |
|---|---|---|
| Polymer 1 | 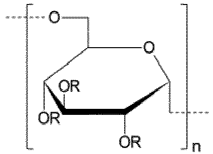 | |
| | Mw (Dextran) = 40 kg/mol | |
| | n = 205 | |
| | R = H or | 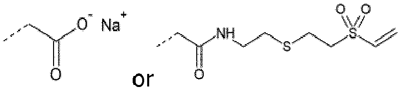 |
| | DS₂ = 2.1 | |
| | DS₁ = 0.25 | |

### Polymer 1

### Polymer 1.1:

65 g (0.4 mol of glucoside units, 1.2 mol of hydroxyl functional groups) of dextran having a weight-average molar mass of 40 kg/mol (Pharmacosmos, degree of polymerization n = 205), are dissolved in water (285 g/L) at 30°C, then NaBH₄ (74 mg, 1.95 mmol) is added and the mixture is stirred at 30°C for 2 h. To this solution is added sodium chloroacetate (140 g, 1.2 mol) and the mixture is heated at 65°C for 1 h. 10 N NaOH (200 mL, 2 mol) is then slowly added over 1.5 h and the mixture is stirred at 65°C for 1 h. The mixture is diluted with water (120 mL), cooled to room temperature, neutralized with acetic acid and then purified by ultrafiltration on PES membrane (MWCO 5 kDa) against phosphate buffer pH 7, then water. The polymer 1.1 concentration of the final solution is determined by dry extract, and then an acid/base assay is carried out in order to determine the degree of substitution with methyl carboxylate.

According to the dry extract: [polymer 1.1] = 50.6 mg/g

According to the acid/base assay, degree of substitution with methylcarboxylate (DS₂) = 1.2

### Polymer 2.1:

90 g (0.35 mol of glucoside units) of freeze-dried polymer 1.1 are dissolved in water (260 g/L) at 65°C, then sodium chloroacetate (204 g, 1.75 mol) is added and the mixture is maintained at 65°C for 1 h. 10 N NaOH (175 mL, 1.75 mol) is then slowly added over 1 h and the mixture is stirred at 65°C for a further 1 h. Another portion of sodium chloroacetate (122 g, 1.05 mol) is then added and the mixture is maintained at 65°C for 0.5 h. 10 N NaOH (105 mL, 1.05 mol) is then slowly added over 1 h and the mixture is stirred at 65°C for a further 1 h. The mixture is diluted with water, cooled to room temperature, neutralized with acetic acid and then purified by ultrafiltration on PES membrane (MWCO 5 kDa) against phosphate buffer pH 7, then water. The polymer 2.1 concentration of the final solution is determined by dry extract, and then an acid/base assay is carried out in order to determine the degree of substitution with methyl carboxylate.

According to the dry extract: [polymer 2.1] = 48.4 mg/g

According to the acid/base assay, degree of substitution with methylcarboxylate (DS₂) = 2.1

### Polymer 1

To 207 g of the solution of polymer 2.1 (48.4 mg/g, DS2 = 2.1, 10.0 g, 30.28 mmol of glucoside units), 2-hydroxypyridine 1-oxide (HOPO) (1.68 g, 15.14 mmol) is added and the mixture is cooled to 4°C. To this solution are added 2-[[2-(ethenylsulfonyl)ethyl]thio]ethanamine hydrochloride (VS) (2.10 g, 9.08 mmol) (synthesized according: S. A. Stewart et al., Soft Matter, 2018, 14, 8317), Et3N (1.27 mL, 9.08 mmol) and N-ethyl-N'-(3-dimethylaminopropyl)carbodiimide hydrochloride (EDC) (2.90 g, 15.14 mmol) and the reaction mixture is stirred between 4°C and 25°C for 2 h. Two additional additions of EDC (2.90 g, 15.14 mmol) are performed every 2 h. The mixture is diluted with NaCl (9 g/L in water), then purified by ultrafiltration on PES membrane (MWCO 5 kDa) against NaCl (9 g/L in water), carbonate buffer pH 9-10, NaCl (9 g/L in water), phosphate buffer pH 7, NaCl (9 g/L in water), and then water. The polymer 1 concentration of the final solution is determined by dry extract, and the degree of substitution with vinyl sulfone is determined by 1H NMR in D2O. The final solution is stored at -20°C.

According to the dry extract: [polymer 1] = 21.3 mg/g

According to 1H NMR (D2O), degree of substitution with vinyl sulfone (DS1) = 0.25

### Example A2 : Polyethylene glycol derivatives comprising at least two reactive functions

Commercial Polyethylene glycol (PEG) derivatives functionalized with reactive functions were purchased. The reactive functions include thiols ("PEG-SH"),. Multi-arms homofunctional having different molecular weights and bearing different functions were used and are shown in the following Table 2.

**Table 2: List of commercial PEG derivatives used**

| **PEG** | **Chemical Name *(Supplier)*** | **Structure** |
|---|---|---|
| PEG-SH-1 | 4-arm PEG-thiol, pentaerythritol core *(Jenkem; Ref. 4ARM-SH-20K*) | 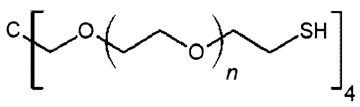 |
| | | Mn (PEG) = 20 kg/mol |
| | | n = 114 |
| PEG-SH-2 | 4-arm PEG-thiol, pentaerythritol core (*NOF*; *Ref. SUNBRIGHT PTE-050SH)* | 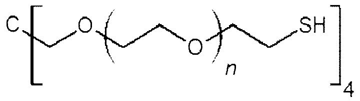 |
| | | Mn (PEG) = 5.2 kg/mol |
| | | n = 30 |

### Part B - PHYSICO-CHEMISTRY

### Example B1: Preparation of suspension of LNG

Levonorgestrel (LNG) powder was purchased from Sigma Aldrich (Saint Quentin Fallavier, France)

A concentrated solution of NaCl was prepared by weighing the appropriate weight of NaCl and adding the appropriate weight of sterile deionized water before sterile filtration (0.22 µm). A concentrated solution of polysorbate 80 was prepared by weighing the appropriate weight of polysorbate 80 and adding the appropriate weight concentration solution of NaCl and sterile deionized water before sterile filtration (0.22 µm). A concentrated suspension of levonorgestrel was prepared by weighing the appropriate weight of levonorgestrel and adding the appropriate weight of sterile concentrated solution of polysorbate 80. The suspension was mixed using vortex.

The suspension of LNG was diluted at 0.7 mg/mL. Microscopy evaluation was carried out using microscope equipped with a x40 lens. The Ferret Diameter was reported.

**Table 3: LNG suspension characterization - NM is Not Measured**

| **Example** | **[LNG] (mg/mL)** | **[PS80] (mg/mL)** | **Size (µm)** |
|---|---|---|---|
| B1-1 | 150 | 5.8 | 2 |
| B1-2 | 50 | 1.9 | NM |

### Example B2: Preparation of solutions of concentrated polysaccharide functionalized with vinyl sulfone (VS) groups

A concentrated polysaccharide solution was prepared by weighing the appropriate weight of a sterile freeze-dried polysaccharide obtained according to part A1 and adding the appropriate weight of sterile deionized water. The solution was placed on an orbital shaker overnight at 70 rpm for complete solubilization. The mass concentration of the solution of polysaccharide (mg/g) was determined by dry extract. The volume concentration of the solution of polysaccharide (mg/mL) was determined by density measurements, weighing three times 100 µL of solution. The sterile solution was frozen at -20°C until being used.

### Example B3: Preparation of solutions of concentrated PEG derivative

A concentrated solution of PEG derivative (from the list according to table 2) was prepared by weighing the appropriate weight of a PEG derivative powder and adding the appropriate weight of sterile deionized water. The solution was placed on roller shaker at 15 rpm for 2 h for complete solubilization before sterile filtration (0.22 µm). The mass concentration of the PEG solution (mg/g) was determined by dry extract. The volume concentration of the PEG solution (mg/mL) was determined by density measurements, weighing three times 100 µL of solution. The sterile solution was frozen at -20°C until being used.

### Example B4: Hydrogels preparation

The hydrogels were prepared from LNG concentrated suspension, polysaccharide and PEG derivatives concentrated sterile solutions prepared according to example B1, B2 and B3, respectively, and made an aseptic environment.

A concentrated suspension of LNG in PEG derivatives solution was prepared by weighing the appropriate weight of concentrated suspension of LNG and adding the appropriated weight of concentrated sterile PEG derivatives solution and sterile deionized water.

Polysaccharide sterile solution and suspension of LNG in PEG derivatives solution were adjusted with a concentrated NaCl solution to obtain isotonic stock solutions (300 mOsm/kg) and equilibrated either at room temperature (20-25°C).

Polysaccharide sterile solution was buffered with 10mM of 2-(N-morpholino)ethanesulfonic acid (MES) for pH 6 or tris(hydroxymethyl)aminomethane (Tris) for pH 8 and suspension of LNG in PEG derivatives solutions was buffered with 10mM of MES for pH 6, before sterile filtration (0.22 µm).

A concentrated suspension of LNG in PEG derivative was added to a concentrated solution of polysaccharide in a 2 mL Eppendorf at room temperature (20-25°C). The volume ratio of the suspension of LNG in PEG to the polysaccharide solution was 70:30 (%:%) or 50:50 (%:%) depending on hydrogel composition.

### Example B4A: Hydrogels preparation for implantation

Using a process similar to the one used for the preparation of hydrogel B4, the suspension of LNG in PEG solution and the polysaccharide solution were mixed with a pipette and a controlled volume of this reconstituted solution was introduced in a circular silicone isolator adhering to a glass slide Crosslinking leading to gelation was carried out for 1 h at 37°C. The hydrogel was unmolded then was immersed in 5 mL of the PBS solution overnight at 37°C. The hydrogel piece was then stored in 5 mL of PBS solution at 4°C until being used.

**Table 4: Molded hydrogel geometries conditions**

| **Geometry** | **Silicone Isolator Diameter (mm)** | **Silicone Isolator Thickness (mm)** | **Hydrogel Volume (µL)** |
|---|---|---|---|
| B4A-1 | 9 | 1.6 | 100 |

### Example B4B: Hydrogels preparation for injection

Using a process similar to the one used for the preparation of hydrogel B4, the suspension of LNG in PEG solution and the polysaccharide solution were mixed with a pipette and a controlled volume of this reconstituted solution was inserted in a syringe in order to be immediately injected.

### Example B5: Hydrogel compositions

Different hydrogel compositions were prepared according to the protocol described in Example B4A and B4B. Concentrations of the two reactive groups (vinylsulfone (VS) on polymers reacting with thiol (SH) and polymers (polysaccharide) and PEG derivatives) correspond to the final concentration after mixing.

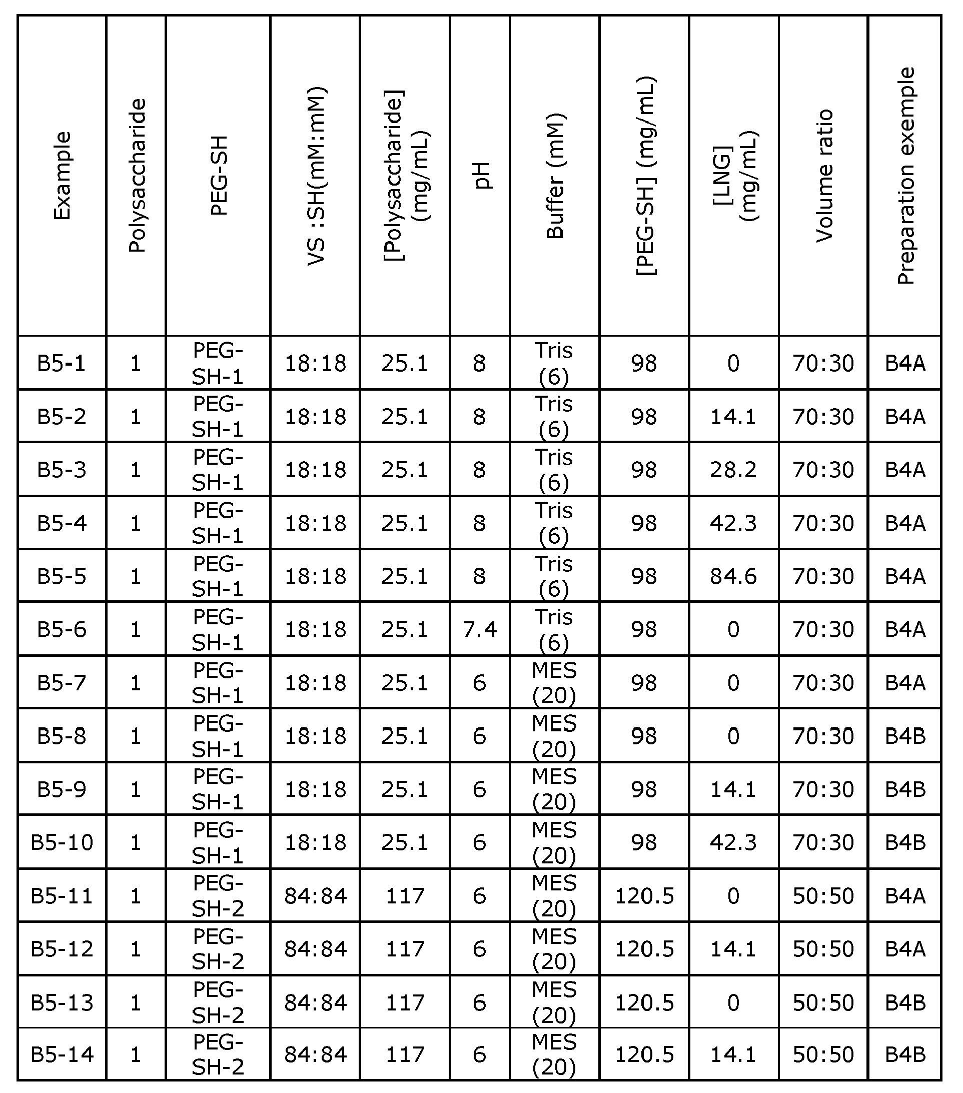

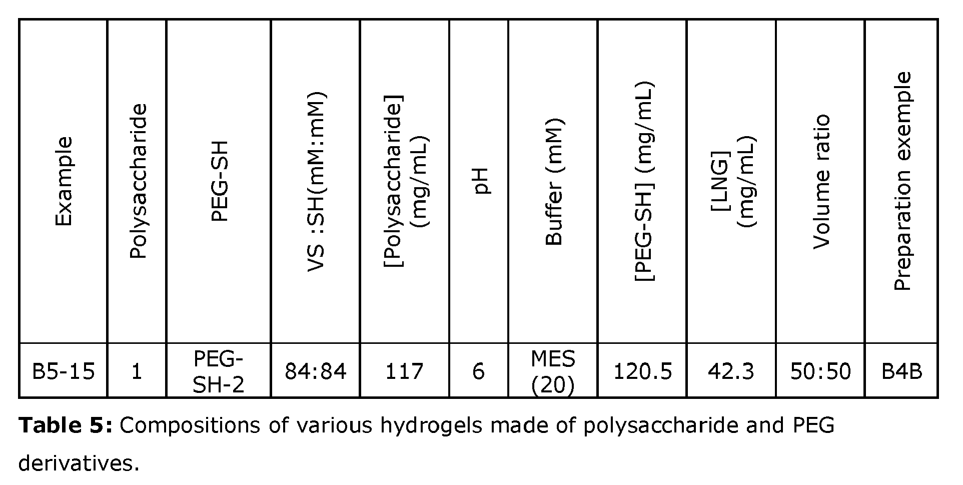

The gels obtained here above presents the following chemical structure.

**Table 5a: Structures of the hydrogels B5-1 to B5-15**

| **Hydrogels (concentration in reactive species (mM) / [LNG] (mg/mL))** | **Structure** | |
|---|---|---|
| B5-1 (18 / 0) | 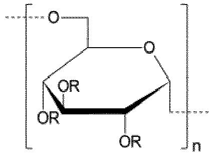 | |
| B5-2 (18 / 14.1) | | |
| B5-3 (18 / 28.2) | | |
| B5-4 (18 / 42.3) | | |
| B5-5 (18 / 84.6) | Mw (Dextran) = 40 kg/mol | |
| B5-6 (18 / 0) | n = 205 | |
| B5-7 (18 / 0) | | |
| B5-8 (18 / 0) | R = H or 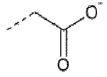 or 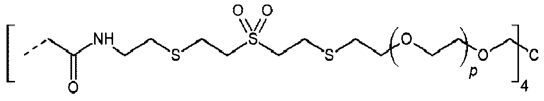 | |
| B5-9 (18 / 14.1) | | |
| B5-10 (18 / 42.3) | | |
| | p = 114 | |
| | DS₂ (methylcarboxylate) = 2.1 | |
| | DS₁ (sulfone derivative) = 0.25 | |

| **Hydrogels (concentration in reactive species (mM) / [LNG] (mg/mL))** | **Structure** | |
|---|---|---|
| B5-11 (84 / 0) | 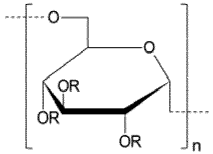 | |
| B5-12 (84 / 14.1) | | |
| B5-13 (84 / 28.2) | | |
| B5-14 (84 / 14.1) | | |
| B5-15 (84 / 42.3) | Mw (Dextran) = 40 kg/mol | |
| | n = 205 | 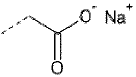 |
| | R = H or | 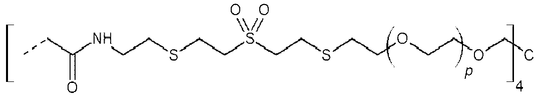 or |
| | p = 30 | |
| | DS₂ (methylcarboxylate) = 2.1 | |
| | DS₁ (sulfone derivative) = 0.25 | |

### Exemple B6 : Viscosity of precursors concentration solution

Flow curve measurement was carried out with a rotational rheometer (AR2000, TA instrument) equipped with a cone plate geometry. Logarithmic sweep tests were carried out at 25°C, with shear rate ranging from 10 to 1000 s⁻¹. The dynamic viscosity was reported as function of shear rate. The precursor solutions present Newtonian fluid behavior. The dynamic viscosity is represented by the mean viscosity in the range tested.

**Table 6: Results of hydrogel viscosity characterization.**

| **Example** | **Hydrogel composition** | **Viscosity of formulation before cross-linking mPa.s)** |
|---|---|---|
| B6-1 | B5-8 | 16.5 |
| B6-2 | B5-13 | 40.5 |

The hydrogels according to the invention exhibit a viscosity suitable for injection thought 20-27G needle and patient compliance.

### Example B7: Hydrogel rheological characterization

Oscillatory shear test was carried out with a rotational rheometer (AR2000, TA instrument) equipped with a cone plate geometry. The cross-linking leading to gelation was done "in situ", meaning that drops of polysaccharide and PEG derivative concentrated solutions (containing or not LNG suspension) were introduced between cone one and plate and mixed by rotation of the geometry before starting oscillations measurements. Oscillation time sweep tests were carried out at 37°C, with a constant strain of 0.1% and constant oscillation frequency of 1 Hz. The storage modulus G' (i.e. elastic modulus) and Tan δ (ratio G'/G") values were reported at 3600 s in the plateau region of the measure of (G',G") as a function of time.

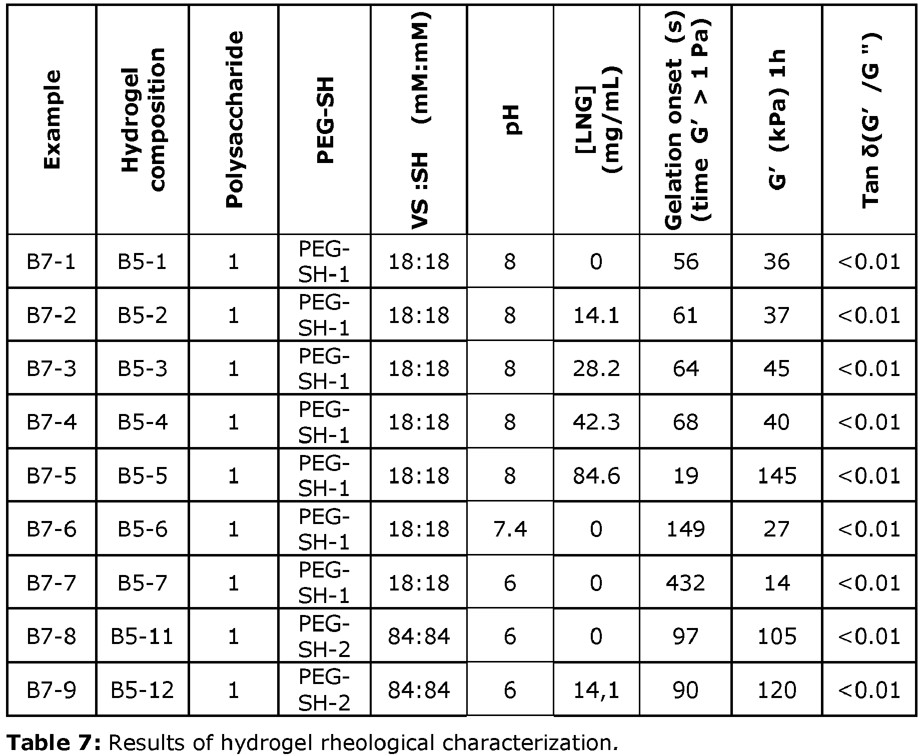

Hydrogels present low values of Tan δ, meaning that G' was much higher than G" which is a typical property of chemically cross-linked hydrogels behaving as solid elastic materials (see Polysaccharide Hydrogels: Characterization and Biomedical Applications, 2016 Pan Stanford Publishing Pte. Ltd.; Chapter 3, page 97).

Increasing the pH was a way to accelerate the cross-linking leading to gelation of hydrogels prepared from polysaccharide bearing VS groups and PEG-SH. For example, going from pH 6 to pH 8 leads to a faster gelation.

This can allow a fine tuning of the gelation speed which could be convenient as fast gelation could be beneficial to avoid gel spreading whereas slow gelation could be beneficial for polymer mix casting or injection before gelation.

### Example B8: Hydrogel swelling and water content

The hydrogel piece was weighed right after unmoulding (wo) and after overnight swelling (w_{overnight}) in the PBS solution. The swelling ratio was defined as the mass ratio w_{overnight}/w₀. The water content of the hydrogel was deducted from the measurement of hydrogel mass in the swollen state and the control of polymer precursors concentrations implemented to synthetize the hydrogel.

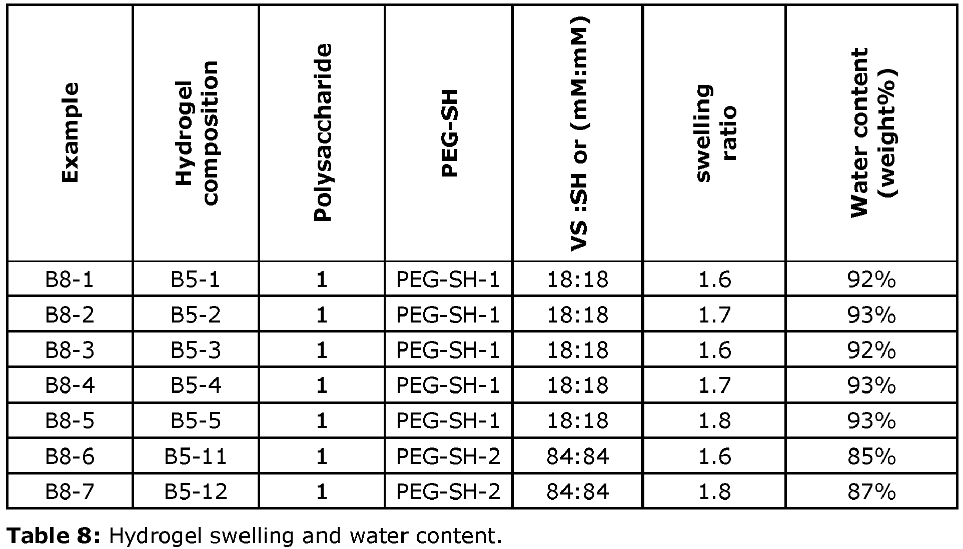

Hydrogels contain high water content. Water content varies depending on polymer precursors structures and concentrations.

### Example B9: Determination of mechanical resistance of the hydrogels.

For compression, a swollen cylindrical hydrogel piece as described in Example B5 was introduced in a flat glass crystallizer and immersed in PBS. The uniaxial compression was done in PBS at 20-25°C by using a universal mechanical tester apparatus (Zwickroell) equipped with flat compression plates, at a speed of 0.2 mm/min. Initial thickness of the sample was determined from the contact of the plate with the hydrogel, when the force starts to increase. The deformation is defined by the ratio of the compression displacement (mm) and the initial thickness (mm). The deformation at break was determined from the force/displacement curve. The break is defined when a decrease of the force versus displacement was observed.

The hydrogels according to the invention exhibit very good mechanical resistances features combining deformability and stiffness suitable for implantation, conservation of mechanical properties in tissue and removability.

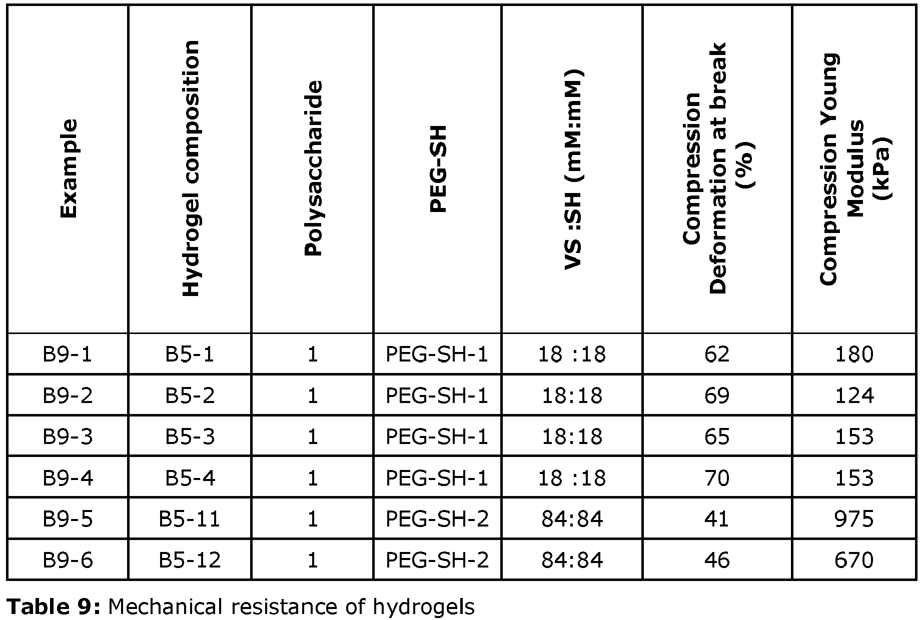

### Example B10A: In vitro release experiment of LNG from suspension and from hydrogel

Suspension of LNG described in Example B1 was introduced in a glass bottle and immersed with 100 mL of a buffer solution of PBS (1×)/ BSA (20 g/L) at pH 7.4 and supplemented with 1% (v/v) Pen/Strep solution. The experiment was conducted at 37°C under constant orbital shaking (80). The entire media was centrifuged at 10000 g during 15 minutes at 20°C at different timepoint. 80 mL of buffer were sampled and replaced by fresh buffer. The concentration of LNG in sample were determined by HPLC. The cumulative fraction of LNG released at each time point corresponding to the ratio of the cumulative quantity of LNG release to the initial quantity of LNG in the suspension is reported.

Hydrogels (100 µL prior swelling) with encapsulated suspension of LNG as listed in Example B5 were each introduced in a glass bottle and immersed with 100 mL of a buffer solution of PBS (1×)/ BSA (20 g/L) at pH 7.4 and supplemented with 1% (v/v) Pen/Strep solution. The experiment was conducted at 37°C under constant orbital shaking (80-200 rpm). 80 mL of buffer were sampled at different timepoint and replaced by fresh buffer. The concentration of LNG in sample were determined by HPLC. The cumulative fraction of LNG released at each time point corresponding to the ratio of the cumulative quantity of LNG release to the initial quantity of LNG in the unswollen hydrogel is reported.

**Table 9: in vitro release rate of LNG in µg/day**

| | **Released levonorgestrel in µg/day** | | | | | |
|---|---|---|---|---|---|---|
| Example | Composition | 1 day | 2 days | 9 days | 28 days | 56 days |
| B10-1 | Suspension B1-2 | 1126 | 79 | 0 | - | - |
| B10-2 | Hydrogel B5-2 | 49 | 36 | 25 | 14 | 7 |
| B10-3 | Hydrogel B5-3 | 47 | 40 | 32 | 19 | NP |
| B10-4 | Hydrogel B5-4 | 47 | 42 | 37 | 24 | 14 |
| B10-5 | Hydrogel B5-12 | 43 | 34 | 18 | 14 | 8 |

**Table 10: in vitro cumulative release of LNG in %**

| | **Cumulative release of levonorgestrel in %** | | | | | | |
|---|---|---|---|---|---|---|---|
| Example | Composition | 0 | 1 day | 2 days | 9 days | 28 days | 56 days |
| B10-1 | Suspension B1-2 | 11% | 81% | 86% | 86% | - | - |
| B10-2 | Hydrogel B5-2 | 0% | 3% | 6% | 21% | 43% | 59% |
| B10-3 | Hydrogel B5-3 | 0% | 2% | 3% | 12% | 26% | NP |
| B10-4 | Hydrogel B5-4 | 0% | 1% | 2% | 9% | 20% | 30% |
| B10-5 | Hydrogel B5-12 | 0% | 3% | 6% | 19% | 35% | 53% |

These hydrogels allow a slower and longer release of levonorgestrel compared to suspension B1-2. Duration of the release can be modulated through the quantity of levonorgestrel loaded in the hydrogels.

### Example B10B: In vitro release experiment of LNG from hydrogel

Hydrogel's composition B5-9 (100 µL prior swelling) was introduced in a mold dived in a glass bottle containing 100 mL of a buffer solution of PBS (1×)/ BSA (20 g/L) at pH 7.4 and supplemented with 1% (v/v) Pen/Strep solution. The experiment was conducted at 37°C under constant orbital shaking (80 rpm) for 2 hours. The entire volume of buffer was sampled. The concentration of LNG in sample was determined by HPLC. The cumulative fraction of LNG released at 2 hours in the unswollen hydrogel is reported. Hydrogel B5-9 releases around 1% of total during the 2 hours after introduction of the gel precursor in the buffer, showing the absence of burst effect.

### Example B11: Ex vivo subcutaneous administration of hydrogels

Hydrogel's composition B5-8, B5-10 and B5-13 (400 µL prior swelling) were injected subcutaneously in pork belly within 7 sec through 1-mL syringe equipped with a 21-G needle at room temperature. The cross-linking leading to gelation occurred at 37°C.

The hydrogels according to the invention were palpable, homogenous, non-diffused and removable from subcutaneous tissue

## Claims

1. Cross-linked dextran polymer Dx bearing anionic groups wherein the at least divalent radical L(-)i is covalently bound to the dextran polymer backbone with i-W-radicals, wherein,
- L(-)ᵢ is a linear or branched polyether
- i is the valence of L and the number of-W-radicals bound to the dextran polymer and is an integer comprised from 2 to 8 (2 ≤ i ≤ 8),
- -W- is a radical comprising at least a radical alkyl linear or branched and optionally comprising heteroatoms such as oxygen, nitrogen or sulfur, aromatic cycles, polyether derivatives and that does not comprise two or more alpha aminoacid residues in particular linked by peptidic bond(s).

2. Cross-linked dextran polymer according to claim 1, wherein the central-linker L(-)ᵢ is a branched PEG radical which possesses at most 8 arms.

3. Cross-linked dextran polymer according to claim 1, wherein the anionic groups are chosen among alkyl carboxylate anions, sulphate anions, or sulfonate anions, or phosphate anions, or phosphonate anions.

4. Cross-linked dextran polymer according to claim 1, wherein the anionic groups are chosen among anionic groups of Formula II: 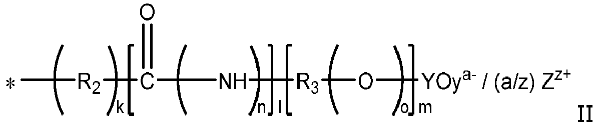 Wherein:
• ^{∗} represents the link to the O atoms of the dextran to form an ether function.
• y=2 or 3.
• When y=2, alkyl carboxylate derivatives, then:
∘ Y=C and a=1.
∘ k=1, l=0 and m=0.
∘ R2=Alkyl.
• When y=3, anionic group, then:
∘ Y=S and a=1, or Y=P and b=2.
∘ k=0 or 1.
∘ l=0 or 1.
∘ m=0 or 1.
∘ n=1 or 2, in particular n = 1.
∘ o=0 or 1.
∘ if l=1 then m=1.
∘ R₃=linear, branched, or cyclic alkyl which may contain one heteroatom such as nitrogen, or aromatic, or PEG.
∘ R₂=Alkyl.
• And, Z is a counter ion, which can be an alkali metal and z=1, or which can be an alkaline earth metal and z=2.

5. Cross-linked dextran polymer according to claim 1, wherein -W- is chosen among the radicals of formula IV. 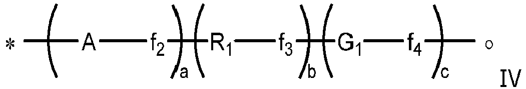 Wherein
• is the site of f₁ and ° represents the site of attachment with L
• a is an integer equal to 0 or 1.
• b is an integer equal to 0 or 1.
• c is an integer equal to 0 or 1.
• i is an integer comprised from 2 to 8, (2 ≤ i ≤ 8).
• In one embodiment a=0, f₁ is an ether function, or a carbamate function.
• In one embodiment a=1,
∘ the divalent radical -A- is a linear, -(CH₂)ₙ₁- with n₁ an integer comprised from 1 to 7 (1 ≤ n₁ ≤ 7), branched, or cyclic alkyl derivative, . It may also be branched by at least one hydroxyl group, -CH₂-CH(OH)-(CH₂)ₙ₂- with n₂ an integer comprised from 1 to 5 (1 ≤ n₂ ≤ 5); f₁ is an ether function, or a carbamate function, and f₂ is an amide function.
▪ Or,
∘ the divalent radical -A- is a linear polyether (PEG) derivative; f₁ is an ether function, or a carbamate function, and f₂ is an amide function.
▪ Or,
∘ in another embodiment the divalent radical -A- is a 4-Alkyl-1,4-triazole derivative or a 4-PEG-1,4-triazole derivative; f₁ is an ether function, or a carbamate function, and f₂ is a carbon-nitrogen covalent bond.
▪ Or,
∘ in another embodiment the divalent radical -A- is a 1-Alkyl-1,4-triazole derivative or a 1-PEG-1,4-triazole derivative; f₁ is an ether function, or a carbamate function, and f₂ is a carbon-aromatic carbon covalent bond.
• The divalent radical -R₁- is a linear, branched, or cyclic alkyl derivative, and/or an aromatic derivative, and/or a polyether (PEG) derivative, which can contain heteroatoms such as nitrogen, oxygen, or sulphur.
∘ If b=0, then f₁ is an ether function, or a carbamate function.
∘ If b=1, then f₁ is an ether function, or a carbamate function, and f₃ is an amide function, or an amine function, or an ether function, or a thioether function, or a carbamate function, or a carbon-nitrogen covalent bond, or a carbon-aromatic carbon covalent bond.
• The divalent radical -G₁- is a linear, branched, or cyclic alkyl derivative, or an aromatic derivative, which can contain heteroatoms such as: at most 5 nitrogen atoms, at most 10 oxygen atoms, at most 5 sulphur atoms, or at most one phosphorus atom. In a preferred embodiment, -G₁- is a succinimide derivative, or an alkyl sulfone derivative which can contain one heteroatom such as oxygen or sulphur, or an ethyl amide derivative, or a 1,4-triazole derivative, or a multicycle derivative from a Diels-Alder reaction, or an aromatic phosphine derivative created by a Staudinger ligation, or a cysteine derivative coming from a Native Chemical Ligation.
∘ If c=0, then f₁, is an ether function, or a carbamate function.
∘ If c=1, then f₁, is an ether function, or a carbamate function, and f₄ is an amine function, or an amide function, or a carbamate function, or a thioether function, or an ether function, or a carbon-nitrogen covalent bond, or carbon-aromatic carbon covalent bond.

6. Dextran polymers of formula VIII before the crosslinking reaction. 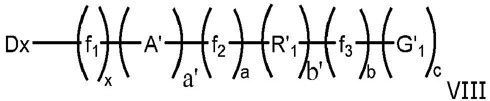 Wherein
• f₁, f₂, f₃, f₄, Dx are defined as in claim IV if none of a, a', b and b' are not equal to 0,
• and
• x equal 0 or 1
• if a, a', b and b' are equal to 0, x is equal to 0 and Dx is a dextran polymer backbone is according to formula III, wherein R is chosen among -H or a anionic group of formula II,
• if one of b' and c is not equal to 0 -A' is A as defined above,
• if b', b and c are equal to 0, a is equal to 0 and A' is the precursor of A before the crosslinking reaction.
• if c is not equal to 0, R'₁ is R₁ as defined above and G'₁ is the precursor of G₁.
• if c is equal to 0, b is equal to 0 and R'₁ is the precursor of R₁ before the crosslinking , reaction.

7. Process to synthetize a cross-linked dextran polymer according to the invention, into the form of a hydrogel, comprising the steps of:
a) preparation of a sterile solution comprising a dextran bearing anionic groups of formula II and at least two precursors of -W-
b) preparation of a sterile solution of a precursor of L(-)ᵢ
c) addition of the sterile solution obtained from step b) to the solution obtained from step a),
d) the addition being directly done in a mould or the solutions are introduced into a mould after being mixed,
e) cross-linking and gelation, for example at room temperature (20-25°C) or at 37°C,
f) unmoulding and swelling to obtain an hydrogel.

8. Process to prepare a hydrogel comprising an API comprising the steps of:
a) preparation of a sterile solution comprising a dextran bearing anionic groups of formula II and at least two precursors of -W-,
b) preparation of a sterile solution of a precursor of L(-)ᵢ,
c) preparation of a suspension of an API,
d) mixing the API suspension obtained from step c) and the solution obtained from the step b) or a),
e) addition of the sterile solution obtained from step a or b which is not used in step d) to the solution obtained from step d),
f) the addition of step e) being either done directly in a mould or the solutions are introduced into a mould after being mixed,
g) cross-linking and gelation reaction at room temperature (20-25°C),
h) unmoulding and swelling to obtain an hydrogel comprising an API.

9. Hydrogel comprising the crosslinked dextran polymer according to any one of the preceding claims.

10. Hydrogel according to claim 9, **characterized in that** it further comprises an API.

11. Therapeutic use of the hydrogel according to any one of claims 9 to 10, for treating a disorder or disease in a mammal.

12. Implant comprising the hydrogel according to any one of claims 9 to 10
